# EUROPEAN PATENT SPECIFICATION

(11) **EP 2 294 221 B1**
(45) Date of publication and mention of the grant of the patent: **16.12.2015**
(21) Application number: 09761804.5
(22) Date of filing: 15.06.2009
(51) Int. Cl.: C12Q 1/68

(54) **ACE2 AS A TARGET GENE FOR THE MOLECULAR IDENTIFICATION OF YEAST AND FUNGAL SPECIES**
ACE2 ALS ZIELGEN FÜR DIE MOLEKULARE IDENTIFIZIERUNG VON HEFE- UND PILZSPEZIES
ACE2 EN TANT QUE GÈNE CIBLE POUR L'IDENTIFICATION MOLÉCULAIRE D'ESPÈCES DE LEVURE ET FONGIQUES

(30) Priority: 13.06.2008 IE 20080486
(43) Date of publication of application: 16.03.2011
(73) Proprietor: National University of Ireland, Galway, Galway (IE)
(72) Inventor: BARRY, Thomas Gerard, Co. Galway (IE); SMITH, Terry James, Galway (IE); MAHER, Majella, Co. Galway (IE); JANKIEWICZ, Marcin, Co. Clare (IE); O'CONNOR, Louise, Galway (IE); TUITE, Nina, Galway (IE); LAHIFF, Sinead, Galway (IE)
(74) Representative: Gates, Marie Christina Esther
(86) International application number: PCT/EP2009/057344
(87) International publication number: WO 2009/150243

(56) References cited:
- BE-A3- 1 014 556
- US-A- 6 017 699
- DATABASE EMBL [Online] 10 October 2004 (2004-10-10), "Sequence 4974 from patent US 6747137." XP002546785 retrieved from EBI accession no. EMBL:AR549843 Database accession no. AR549843 -& US 2007/027309 A1 (WEINSTOCK KEITH G [US] ET AL) 1 February 2007 (2007-02-01) cited in the application
- MACCALLUM DONNA M ET AL: "Different consequences of ACE2 and SWI5 gene disruptions for virulence of pathogenic and nonpathogenic yeasts." INFECTION AND IMMUNITY SEP 2006, vol. 74, no. 9, September 2006 (2006-09), pages 5244-5248, XP002546783 ISSN: 0019-9567
- KELLY MARY T ET AL: "The Candida albicans CaACE2 gene affects morphogenesis, adherence and virulence" MOLECULAR MICROBIOLOGY, vol. 53, no. 3, August 2004 (2004-08), pages 969-983, XP002546784 ISSN: 0950-382X

## Description

### Field of the Invention

The present invention relates to nucleic acid primers and probes for use in the identification of one or more yeast species. More specifically the invention relates to the Ace2 gene, the corresponding RNA, specific probes, primers and oligonucleotides related thereto and their use in diagnostic assays to detect and / or discriminate between yeast species, as defined in the appended claims.

### Background to the Invention

Yeast and fungal infections represent a major cause of morbidity and mortality among immunocompromised patients. The number of immunocompromised patients at risk of yeast and fungal infection continues to increase each year, as does the spectrum of fungal and yeast agents causing disease. Mortality from fungal infections, particularly invasive fungal infections, is 30% or greater in certain risk groups. The array of available anti-fungal agents is growing; however, so too is the recognition of both intrinsic and emerging resistance to antifungal drugs. These factors are contributing to the increased need for cost containment in laboratory testing and have led to laboratory consolidation in testing procedures.

Invasive fungal infections are on the increase. In 2003, it was estimated that there were 9 million at risk patients of which 1.2 million developed infection. *Candida* species now rank as the most prominent pathogens infecting immunosupressed patients. In particular, infections are common in the urinary tract, the respiratory system and the bloodstream, at the site of insertion of stents, catheters and orthopaedic joints. Approximately 10% of the known *Candida* spp. have been implicated in human infection. Invasive candidiasis occurs when candida enters the bloodstream and it is estimated to occur at a frequency of 8/100,000 population in the US with a mortality rate of 40%. *Candida albicans* is the 4^{th} most common cause of bloodstream infection. Emerging mycoses agents include *Fusarium, Scedosporium, Zygomycetes* and *Trichosporon* spp. ("Stakeholder Insight: Invasive fungal infections", Datamonitor, Jan 2004).

Immunocompromised patients including transplant and surgical patients, neonates, cancer patients, diabetics and those with HIV/AIDs are at high risk of developing invasive fungal infections (Datamonitor report: Stakeholder opinion -Invasive fungal infections, options outweigh replacements 2004). A large number of severe cases of sepsis are reported each year. Despite improvements in its medical management, sepsis still constitutes one of the greatest challenges in intensive care medicine. Microorganisms (bacteria, fungi and yeast) responsible for causing sepsis are traditionally detected in hospital laboratories with the aid of microbiological culture methods with poor sensitivity (25-82%), which are very time-consuming, generally taking from two to five days to complete, and up to eight days for the diagnosis of fungal infections.

Definitive diagnosis of infection caused by yeast or fungus is usually based on either, the recovery and identification of a specific agent from clinical specimens or microscopic demonstration of fungi or yeasts with distinct morphological features. However, there are numerous cases where these methods fail to provide conclusive proof as to the infecting agent. In these instances, the detection of specific host antibody responses can be used, although again this can be affected by the immune status of the patient. Time is critical in the detection and identification of bloodstream infections typically caused by bacteria, yeast or fungi. Effective treatment depends on finding the source of infection and making appropriate decisions about antibiotics or antifungals quickly and efficiently.

Fungal and yeast nucleic acid based diagnostics have focused heavily on the ribosomal RNA (rRNA) genes, RNA transcripts, and their associated DNA / RNA regions. The rRNA genes are highly conserved in all fungal species and they also contain divergent and distinctive intergenic transcribed spacer regions. Ribosomal rRNA comprises three genes: the large sub-unit gene (28S), the small sub-unit gene (18S) and the 5.8S gene. The 28S and 18S rRNA genes are separated by the 5.8S rRNA and two internal transcribed spacers (ITS1 and ITS2). Because the ITS region contains a high number of sequence polymorphisms, numerous researchers have concentrated their efforts on these as targets (Atkins and Clark, 2004). rRNA genes are also multicopy genes with >10 copies within the fungal genome.

A number of groups are working on developing new assays for fungal and yeast infections. US2004044193 relates to, amongst a number of other aspects, the transcription factor CaTEC1 of *Candida albicans*; inhibitors thereof, and methods for the diagnosis and therapy of diseases which are connected with a Candida infection; and also diagnostic and pharmaceutical compositions which contain the nucleotide sequences, proteins, host cells and/or antibodies. WO0183824 relates to hybridization sequences, proteins, host cells and/or antibodies. BE 1014556 relates to a mixture of oligonucleotides, useful for specific amplification of Candida albicans genes, particularly for diagnosis and identification of therapeutic agents. WO0183824 relates to hybridization assay probes and accessory oligonucleotides for detecting ribosomal nucleic acids from *Candida albicans* and/or *Candida dubliniensis.* US6017699 and US5426026 relate to a set of DNA primers, which can be used to amplify and speciate DNA from five medically important Candida species. US 6747137 discloses sequences useful for diagnosis of Candida infections. EP 0422872 and US 5658726 disclose probes based on 18S rRNA genes, and US 5958693 discloses probes based on 28S rRNA, for diagnosis of a range of yeast and fungal species. US 6017366 describes sequences based on chitin synthase gene for use in nucleic acid based diagnostics for a range of Candida species.

It is clear though, that development of faster, more accurate diagnostic methods are required, particularly in light of the selection pressure caused by modem anti-microbial treatments which give rise to increased populations of resistant virulent strains with mutated genome sequences. Methods that enable early diagnosis of microbial causes of infection enable the selection of a specific narrow spectrum antibiotic or antifungal to treat the infection (Datamonitor report: Stakeholder opinion -Invasive fungal infections, options outweigh replacements 2004; Datamonitor report: Stakeholder Opinion-Sepsis, under reaction to an overreaction, 2006).

Only after pathogens are correctly identified can targeted therapy using a specific antibiotic or antifungal begin. Many physicians would like to see the development of better *in vitro* amplification and direct detection diagnostic techniques for the early diagnosis of yeast and fungi ("*Stakeholder Insight: Invasive fungal infections",* Datamonitor, Jan 2004). The present invention provides novel fungal and yeast nucleic acid targets for application in Nucleic Acid Diagnostics (NAD) tests. These are rapid, accurate diagnostic tests for clinically significant bacterial and fungal pathogens for bioanalysis applications in the clinical sector.

Ace2 is a DNA-binding cell cycle regulated transcription factor. Ace2 functions similarly to the transcription factor SW15, yet they activate distinct genes. The translated Ace2 protein is present in the nucleus in early G1 phase of the cell cycle and thus, specifically activates the expression of genes in the G1 phase. In particular, it activates the CTS1 gene. CTS1 is a chitinase-encoding gene required to degrade the cell wall between parent and daughter cells during cytokinesis. There are 7 Ace2 sequences publicly available in the NCBI GenBank database including 5 *Candida spp.* sequences. There are no published Ace2 sequences available for *Aspergillus spp.* The *spp.* equivalent to base pair position 1736 to 2197 in *C*. *albicans.* This region of the Ace2 gene has an application for the molecular identification and / or discrimination of yeast species.

### Definitions

"Synthetic oligonucleotide" refers to molecules of nucleic acid polymers of 2 or more nucleotide bases that are not derived directly from genomic DNA or live organisms. The term synthetic oligonucleotide is intended to encompass DNA, RNA, and DNA/RNA hybrid molecules that have been manufactured chemically, or synthesized enzymatically *in vitro.*

An "oligonucleotide" is a nucleotide polymer having two or more nucleotide subunits covalently joined together. Oligonucleotides are generally about 10 to about 100 nucleotides. The sugar groups of the nucleotide subunits may be ribose, deoxyribose, or modified derivatives thereof such as OMe. The nucleotide subunits may be joined by linkages such as phosphodiester linkages, modified linkages or by non-nucleotide moieties that do not prevent hybridization of the oligonucleotide to its complementary target nucleotide sequence. Modified linkages include those in which a standard phosphodiester linkage is replaced with a different linkage, such as a phosphorothioate linkage, a methylphosphonate linkage, or a neutral peptide linkage. Nitrogenous base analogs also may be components of oligonucleotides in accordance with the invention.

A "target nucleic acid" is a nucleic acid comprising a target nucleic acid sequence. A "target nucleic acid sequence," "target nucleotide sequence" or "target sequence" is a specific deoxyribonucleotide or ribonucleotide sequence that can be hybridized to a complementary oligonucleotide.

An "oligonucleotide probe" is an oligonucleotide having a nucleotide sequence sufficiently complementary to its target nucleic acid sequence to be able to form a detectable hybrid probe:target duplex under high stringency hybridization conditions. An oligonucleotide probe is an isolated chemical species and may include additional nucleotides outside of the targeted region as long as such nucleotides do not prevent hybridization under high stringency hybridization conditions. Non-complementary sequences, such as promoter sequences, restriction endonuclease recognition sites, or sequences that confer a desired secondary or tertiary structure such as a catalytic active site can be used to facilitate detection using the invented probes. An oligonucleotide probe optionally may be labelled with a detectable moiety such as a radioisotope, a fluorescent moiety, a chemiluminescent, a nanoparticle moiety, an enzyme or a ligand, which can be used to detect or confirm probe hybridization to its target sequence. Oligonucleotide probes are preferred to be in the size range of from about 10 to about 100 nucleotides in length, although it is possible for probes to be as much as and above about 500 nucleotides in length, or below 10 nucleotides in length.

A "hybrid" or a "duplex" is a complex formed between two single-stranded nucleic acid sequences by Watson-Crick base pairings or non-canonical base pairings between the complementary bases. "Hybridization" is the process by which two complementary strands of nucleic acid combine to form a double-stranded structure ("hybrid" or "duplex"). A "fungus" or "yeast" is meant any organism of the kingdom Fungi, and preferably, is directed towards any organism of the phylum Ascomycota.

"Complementarity" is a property conferred by the base sequence of a single strand of DNA or RNA which may form a hybrid or double-stranded DNA:DNA, RNA:RNA or DNA:RNA through hydrogen bonding between Watson-Crick base pairs on the respective strands. Adenine (A) ordinarily complements thymine (T) or uracil (U), while guanine (G) ordinarily complements cytosine (C).

The term "stringency" is used to describe the temperature, ionic strength and solvent composition existing during hybridization and the subsequent processing steps. Those skilled in the art will recognize that "stringency" conditions may be altered by varying those parameters either individually or together. Under high stringency conditions only highly complementary nucleic acid hybrids will form; hybrids without a sufficient degree of complementarity will not form. Accordingly, the stringency of the assay conditions determines the amount of complementarity needed between two nucleic acid strands forming a hybrid. Stringency conditions are chosen to maximize the difference in stability between the hybrid formed with the target and the non-target nucleic acid.

With "high stringency" conditions, nucleic acid base pairing will occur only between nucleic acid fragments that have a high frequency of complementary base sequences (for example, hybridization under "high stringency" conditions, may occur between homologs with about 85-100% identity, preferably about 70-100% identity). With medium stringency conditions, nucleic acid base pairing will occur between nucleic acids with an intermediate frequency of complementary base sequences (for example, hybridization under "medium stringency" conditions may occur between homologs with about 50-70% identity). Thus, conditions of "weak" or "low" stringency are often required with nucleic acids that are derived from organisms that are genetically diverse, as the frequency of complementary sequences is usually less.

'High stringency' conditions are those equivalent to binding or hybridization at 42° C. in a solution consisting of 5xSSPE (43.8g/l NaCl, 6.9 g/l NaH₂PO₄H₂O and 1.85 g/l EDTA, ph adjusted to 7.4 with NaOH), 0.5% SDS, 5xDenhardt's reagent and 100µg/ml denatured salmon sperm DNA followed by washing in a solution comprising 0.1xSSPE, 1.0%SDS at 42° C. when a probe of about 500 nucleotides in length is used.

"Medium stringency' conditions are those equivalent to binding or hybridization at 42° C. in a solution consisting of 5XSSPE (43.8 g/l NaCl, 6.9 g/l NaH₂PO₄H₂O and 1.85 g/l EDTA, pH adjusted to 7.4 with NaOH), 0.5% SDS, 5xDenhardt's reagent and 100 µg/ml denatured salmon sperm DNA followed by washing in a solution comprising 1.0xSSPE, 1.0% SDS at 42° C, when a probe of about 500 nucleotides in length is used.

'Low stringency' conditions are those equivalent to binding or hybridization at 42° C. in a solution consisting of 5xSSPE (43.8 g/l NaCl, 6.9 g/l NaH₂PO₄H₂O and 1.85 g/l EDTA, pH adjusted to 7.4 with NaOH), 0.1% SDS, 5xDenhardt's reagent [50xDenhardt's contains per 500ml: 5g Ficoll (Type 400, Pharamcia), 5 g BSA (Fraction V; Sigma)] and 100 µg/ml denatured salmon sperm DNA followed by washing in a solution comprising 5xSSPE, 0.1% SDS at 42° C, when a probe of about 500 nucleotides in length is used.

In the context of nucleic acid *in-vitro* amplification based technologies, "stringency" is achieved by applying temperature conditions and ionic buffer conditions that are particular to that *in-vitro* amplification technology. For example, in the context of PCR and real-time PCR, "stringency" is achieved by applying specific temperatures and ionic buffer strength for hybridisation of the oligonucleotide primers and, with regards to real-time PCR hybridisation of the probe/s, to the target nucleic acid for *in-vitro* amplification of the target nucleic acid.

One skilled in the art will understand that substantially corresponding probes of the invention can vary from the referred-to sequence and still hybridize to the same target nucleic acid sequence. This variation from the nucleic acid may be stated in terms of a percentage of identical bases within the sequence or the percentage of perfectly complementary bases between the probe and its target sequence. Probes of the present invention substantially correspond to a nucleic acid sequence if these percentages are from about 100% to about 80% or from 0 base mismatches in about 10 nucleotide target sequence to about 2 bases mismatched in an about 10 nucleotide target sequence. In preferred embodiments, the percentage is from about 85% to about 100%. In more preferred embodiments, this percentage is from about 90% to about 100%; in other preferred embodiments, this percentage is from about 95% to about 100%, *e.g.* 95%, 96%, 97%, 98%, 99%, or 100%.

By "sufficiently complementary" or "substantially complementary" is meant nucleic acids having a sufficient amount of contiguous complementary nucleotides to form, under high stringency hybridization conditions, a hybrid that is stable for detection. Substantially complementary to can also refer to sequences with at least 90% identity to, *e.g.,* 95, 96, 97, 98, 99, or 100% identity to, a given reference sequence.

The terms "identical" or percent "identity," in the context of two or more nucleic acids or polypeptide sequences, refer to two or more sequences or subsequences that are the same or have a specified percentage of amino acid residues or nucleotides that are the same (*i.e.,* about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection (*see, e.g.,* NCBI web site at ncbi.nlm.nih.gov/BLAST/ or the like). Such sequences are then said to be "substantially identical." This definition also refers to, or may be applied to, the compliment of a test sequence. The definition also includes sequences that have deletions and/or additions, as well as those that have substitutions. As described below, the preferred algorithms can account for gaps and the like. Preferably, identity exists over a region that is at least about 25 amino acids or nucleotides in length, or more preferably over a region that is 50-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window," as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see, e.g.,* Current Protocols in Molecular Biology (Ausubel et al., eds. 1987-2005, Wiley Interscience)).

A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. BLAST and BLAST 2.0 are used, with the parameters described herein, to determine percent sequence identity for the nucleic acids and proteins of the invention. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (*see* Henikoff & Henikoff, Proc. Natl. Acad. Sci. USA 89:10915 (1989)) alignments (B) of 50, expectation (E) of 10, M=5, N=-4, and a comparison of both strands.

"Nucleic acid" refers to deoxyribonucleotides or ribonucleotides and polymers thereof in either single- or double-stranded form, and complements thereof. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs)

By "nucleic acid hybrid" or "probe:target duplex" is meant a structure that is a double-stranded, hydrogen-bonded structure, preferably about 10 to about 100 nucleotides in length, more preferably 14 to 50 nucleotides in length, although this will depend to an extent on the overall length of the oligonucleotide probe. The structure is sufficiently stable to be detected by means such as chemiluminescent or fluorescent light detection, autoradiography, electrochemical analysis or gel electrophoresis. Such hybrids include RNA:RNA, RNA:DNA, or DNA:DNA duplex molecules.

"RNA and DNA equivalents" refer to RNA and DNA molecules having the same complementary base pair hybridization properties. RNA and DNA equivalents have different sugar groups (i.e., ribose versus deoxyribose), and may differ by the presence of uracil in RNA and thymine in DNA. The difference between RNA and DNA equivalents do not contribute to differences in substantially corresponding nucleic acid sequences because the equivalents have the same degree of complementarity to a particular sequence.

By "preferentially hybridize" is meant that under high stringency hybridization conditions oligonucleotide probes can hybridize their target nucleic acids to form stable probe:target hybrids (thereby indicating the presence of the target nucleic acids) without forming stable probe:non-target hybrids (that would indicate the presence of non-target nucleic acids from other organisms). Thus, the probe hybridizes to target nucleic acid to a sufficiently greater extent than to non-target nucleic acid to enable one skilled in the art to accurately detect the presence of (for example Candida) and distinguish these species from other organisms. Preferential hybridization can be measured using techniques known in the art and described herein.

By "theranostics" is meant the use of diagnostic testing to diagnose the disease, choose the correct treatment regime and monitor the patient response to therapy. The theranostics of the invention may be based on the use of an NAD assay of this invention on samples, swabs or specimens collected from the patient.

### Object of the Invention

It is an object of the current disclosure to provide sequences and/or diagnostic assays to detect and identify one or more yeast species. The current inventors have used the Ace2 gene sequence to design primers and probes that are specific to *Candida* Ace2 genes. Such primers not only allow the detection of yeast and fungal species but also allow distinction between *Candida* species. The current disclosure further provides for primers and probes that allow discrimination between different Candida species.

### Summary of the Invention

The diagnostic kit, nucleic acid molecules and methods of the present invention are defined in the appended claims. The present disclosure provides for a diagnostic kit for detection and identification of yeast species, comprising an oligonucleotide probe capable of binding to at least a portion of the Ace2 gene or its corresponding mRNA. The oligonucleotide probe may have a sequence substantially homologous to or substantially complementary to a portion of the Ace2 gene or its corresponding mRNA. It will thus be capable of binding or hybridizing with a complementary DNA or RNA molecule. The Ace2 gene may be yeast Ace2 gene. The nucleic acid molecule may be synthetic. The kit may comprise more than one such probe. In particular, the kit may comprise a plurality of such probes. In addition the kit may comprise additional probes for other organisms, such as, for example, bacterial species or viruses.

The identified sequences are suitable not only for *in vitro* DNA/RNA amplification based detection systems but also for signal amplification based detection systems. Furthermore, the sequences of the disclosure identified as suitable targets provide the advantages of having significant intragenic sequence heterogeneity in some regions, which is advantageous and enables aspects of the disclosure to be directed towards group or species-specific targets, and also having significant sequence homogeneity in some regions, which enables aspects of the disclosure to be directed towards genus-specific yeast and fungal primers and probes for use in direct nucleic acid detection technologies, signal amplification nucleic acid detection technologies, and nucleic acid in vitro amplification technologies for yeast and fungal diagnostics. The Ace2 sequences allows for multi-test capability and automation in diagnostic assays.

One of the advantages of the sequences of the present disclosure is that the intragenic Ace2 nucleotide sequence diversity between closely related yeast species enables specific primers and probes for use in diagnostics assays for the detection of yeast to be designed. The Ace2 nucleotide sequences, both DNA and RNA can be used with direct detection, signal amplification detection and in *vitro* amplification technologies in diagnostics assays. The Ace2 sequences allow for multi-test capability and automation in diagnostic assays.

The kit may further comprise a primer for amplification of at least a portion of the Ace2 gene. Suitably the kit comprises a forward and a reverse primer for a portion of the Ace2 gene.

The portion of the Ace2 gene may be equivalent to a portion of the region of the gene from base pair position 1736 to base pair position 2197 in *C*. *albicans.* Particularly preferred, are kits comprising a probe for a portion of the Ace2 *C.albicans* gene and / or a probe for a portion of the region of the gene equivalent to base pair position 1736 to base pair position 2197 in *C*. *albicans.* Equivalent regions to base pair position 1736 to base pair position 2197 can be found in other organisms, but not necessarily in the same position.

The probe may preferentially hybridize to a portion of the Ace2 gene sequence selected from the group consisting of SEQ ID NOs: 4-7 and 32-39 or their corresponding mRNA.

The kit may also comprise additional probes. The probe may have a sequence selected from the group consisting of SEQ ID Nos: 3, 30, 31, and a sequence substantially homologous to or substantially complementary to those sequences, which can also act as a probe for the Ace2 gene.

It is desirable that the probe has a sequence as defined by SEQ ID NO: 30.

The kit may comprise at least one forward *in vitro* amplification primer and at least one reverse *in vitro* amplification primer, the forward amplification primer having a sequence selected from the group consisting of SEQ ID Nos: 1,20-24, and sequences being substantially homologous or complementary thereto which can also act as a forward amplification primer, and the reverse amplification primer having a sequence selected from the group consisting of SEQ ID Nos: 2, 25-29, and a sequence being substantially homologous or complementary thereto which can also act as a reverse amplification primer. It is desirable that said forward primer sequence is selected from the group consisting of SEQ ID NOs:21, 22, and 23, and said reverse primer sequence is selected from the group consisting of SEQ ID NOs:27, 28, and 29. The diagnostic kit may be based on direct nucleic acid detection technologies, signal amplification nucleic acid detection technologies, and nucleic acid *in vitro* amplification technologies is selected from one or more of Polymerase Chain Reaction (PCR), Ligase Chain Reaction (LCR), Nucleic Acids Sequence Based Amplification (NASBA), Strand Displacement Amplification (SDA), Transcription Mediated Amplification (TMA), Branched DNA technology (bDNA) and Rolling Circle Amplification Technology (RCAT)), or other *in vitro* enzymatic amplification technologies.

The disclosure also provides a nucleic acid molecule selected from the group consisting of SEQ ID NO.1 to SEQ ID NO. 40 and sequences substantially homologous thereto, or substantially complementary to a portion thereof and having a function in diagnostics based on the Ace2 gene.

The nucleic acid molecule may comprise an oligonucleotide having a sequence substantially homologous to or substantially complementary to a portion of a nucleic acid molecule of SEQ ID NO.1 to SEQ ID NO. 40. The disclosure also provides a method of detecting a target organism in a test sample comprising the steps of:
(i) Mixing the test sample with at least one oligonucleotide probe as defined above under appropriate conditions; and
(ii) Hybridizing under high stringency conditions any nucleic acid that may be present in the test sample with the oligonucleotide to form a probe:target duplex; and
(iii) Determining whether a probe:target duplex is present; the presence of the duplex positively identifying the presence of the target organism in the test sample.

The nucleic acid molecule and kits of the present disclosure may be used in a diagnostic assay to detect the presence of one or more yeast and/or fungal species, to measure yeast and/or fungal titres in a patient or in a method of assessing the efficacy of a treatment regime designed to reduce yeast and/or fungal titre in a patient or to measure yeast and/or fungal contamination in an environment. The environment may be a hospital, or it may be a food sample, an environmental sample e.g. water, an industrial sample such as an in-process sample or an end product requiring bioburden or quality assessment.

The kits and the nucleic acid molecule of the disclosure may be used in the identification and/or characterization of one or more disruptive agents that can be used to disrupt the Ace2 gene function. The disruptive agent may be selected from the group consisting of antisense RNA, PNA, and siRNA.

In some embodiments of the disclosure, a nucleic acid molecule comprising a species-specific probe can be used to discriminate between species of the same genus.

The oligonucleotides of the disclosure may be provided in a composition for detecting the nucleic acids of yeast and fungal target organisms. Such a composition may also comprise buffers, enzymes, detergents, salts and so on, as appropriate to the intended use of the compositions. It is also envisioned that the compositions, kits and methods of the disclosure, while described herein as comprising at least one synthetic oligonucleotide, may also comprise natural oligonucleotides with substantially the same sequences as the synthetic nucleotide fragments in place of, or alongside synthetic oligonucleotides.

The disclosure also provides for an *in vitro* amplification diagnostic kit for a target yeast and/or fungal organism comprising at least one forward *in vitro* amplification primer and at least one reverse *in vitro* amplification primer, the forward amplification primer being selected from the group consisting of one or more of or a sequence being substantially homologous or complementary thereto which can also act as a forward amplification primer, and the reverse amplification primer being selected from the group consisting of one or more of or a sequence being substantially homologous or complementary thereto which can also act as a reverse amplification primer.

The disclosure also provides for a diagnostic kit for detecting the presence of candidate yeast and/or fungal species, comprising one or more DNA probes comprising a sequence substantially complementary to, or substantially homologous to the sequence of the Ace2 gene of the candidate yeast and/or fungal species. The present disclosure also provides for one or more synthetic oligonucleotides having a nucleotide sequence substantially homologous to or substantially complementary to one or more of the group consisting of the Ace2 gene or mRNA transcript thereof, the yeast Ace2 gene or mRNA transcript thereof, the yeast Ace2 gene or mRNA transcript thereof, one or more of SEQ ID NO 1-SEQ ID NO 40.

The nucleotide may comprise DNA. The nucleotide may comprise RNA. The nucleotide may comprise a mixture of DNA, RNA and PNA. The nucleotide may comprise synthetic nucleotides. The sequences of the disclosure (and the sequences relating to the methods, kits compositions and assays of the invention) may be selected to be substantially homologous to a portion of the coding region of the Ace2 gene. The gene may be a gene from a target yeast or fungal organism. The sequences of the disclosure are preferably sufficient so as to be able form a probe:target duplex to the portion of the sequence.

The disclosure also provides for a diagnostic kit for a target yeast or fungal organism comprising an oligonucleotide probe substantially homologous to or substantially complementary to an oligonucleotide of the disclosure (which may be synthetic). It will be appreciated that sequences suitable for use as *in vitro* amplification primers may also be suitable for use as oligonucleotide probes: while it is preferable that amplification primers may have a complementary portion of between about 15 nucleotides and about 30 nucleotides (more preferably about 15-about 23, most preferably about 20 to about 23), oligonucleotide probes of the disclosure may be any suitable length. The skilled person will appreciate that different hybridization and or annealing conditions will be required depending on the length, nature & structure (eg. Hybridization probe pairs for LightCycler, Taqman 5' exonuclease probes, hairpin loop structures etc. and sequence of the oligonucleotide probe selected.

Kits and assays of the disclosure may also be provided wherein the oligonucleotide probe is immobilized on a surface. Such a surface may be a bead, a membrane, a column, dipstick, a nanoparticle, the interior surface of a reaction chamber such as the well of a diagnostic plate or inside of a reaction tube, capillary or vessel or the like.

The target yeast or fungal organism may be selected from the group consisting of *C*. *albicans, C. tropicalis, C. glabrata, C. krusei, C. parapsilosis, C. tropicalis, C. dubliniensis, C. guilliermondii, C. norvegiensis*, *C. famata, C. haemuloni, C. kefyr, C. utilis, C. viswanathii* and *Aspergillus species.*

The target yeast organisms may be a Candida species for the given set of primers already experimentally demonstrated, and more preferably, selected from the group consisting of *C*. *albicans C. tropicalis. C.dubliniensis, C. glabrata, C. krusei, C. parapsilosis, C. guilliermondii, C. norvegiensis, C. famata, C. haemuloni, C. kefyr, C. utilis, C. viswanathii.*

Under these circumstances, the amplification primers and oligonucleotide probes of the disclosure may be designed to a gene specific or genus specific region so as to be able to identify one or more, or most, or substantially all of the desired organisms of the target yeast organism grouping.

The test sample may comprise cells of the target yeast and/or fungal organism. The method may also comprise a step for releasing nucleic acid from any cells of the target yeast or fungal organism that may be present in said test sample. Ideally, the test sample is a lysate of an obtained sample from a patient (such as a swab, or blood, urine, saliva, a bronchial lavage, dental specimen, skin specimen, scalp specimen, transplant organ biopsy, stool, mucus, or discharge sample). The test samples may be a food sample, a water sample an environmental sample, an end product, end product or in-process industrial sample.

The disclosure also provides for the use of any one of SEQ ID NO.1 to SEQ ID NO. 40 in a diagnostic assay for the presence of one or more yeast species. The species may be selected from the group consisting of *C*. *albicans C. tropicalis. C.dubliniensis, C. glabrata, C. krusei, C. parapsilosis, C. guilliermondii, C. norvegiensis, C. famata, C. haemuloni, C. kefyr, C. utilis, C. viswanathii.*

The disclosure also provides for kits for use in clinical diagnostics, theranostics, food safety diagnostics, industrial microbiology diagnostics, environmental monitoring, veterinary diagnostics, bio-terrorism diagnostics comprising one or more of the synthetic oligonucleotides of the disclosure**.** The kits may also comprise one or more articles selected from the group consisting of appropriate sample collecting instruments, reagent containers, buffers, labelling moieties, solutions, detergents and supplementary solutions. The disclosure also provides for use of the sequences, compositions, nucleotide fragments, assays, and kits of the disclosure in theranostics, Food safety diagnostics, Industrial microbiology diagnostics, Environmental monitoring, Veterinary diagnostics, Bio-terrorism diagnostics.

The nucleic acid molecules, composition, kits or methods may be used in a diagnostic nucleic acid based assay for the detection of yeast species.

The nucleic acid molecules, composition, kits or methods may be used in a diagnostic assay to measure yeast or fungal titres in a patient. The titres may be measured *in vitro.*

The nucleic acid molecules, composition, kits or methods may be used in a method of assessing the efficacy of a treatment regime designed to reduce yeast and/or fungal titre in a patient comprising assessing the yeast and/or fungal titre in the patient (by in *vivo* methods or *in vitro* methods) at one or more key stages of the treatment regime. Suitable key stages may include before treatment, during treatment and after treatment. The treatment regime may comprise an antifungal agent, such as a pharmaceutical drug.

The nucleic acid molecules, composition, kits or methods may be used in a diagnostic assay to measure potential yeast and/or fungal contamination, for example, in a hospital.

The nucleic acid molecules, composition, kits or methods may be used in the identification and/or characterization of one or more disruptive agents that can be used to disrupt the Ace2 gene function. Suitable disruptive agents may be selected from the group consisting of antisense RNA, PNA, siRNA.

### Brief description of the Figures

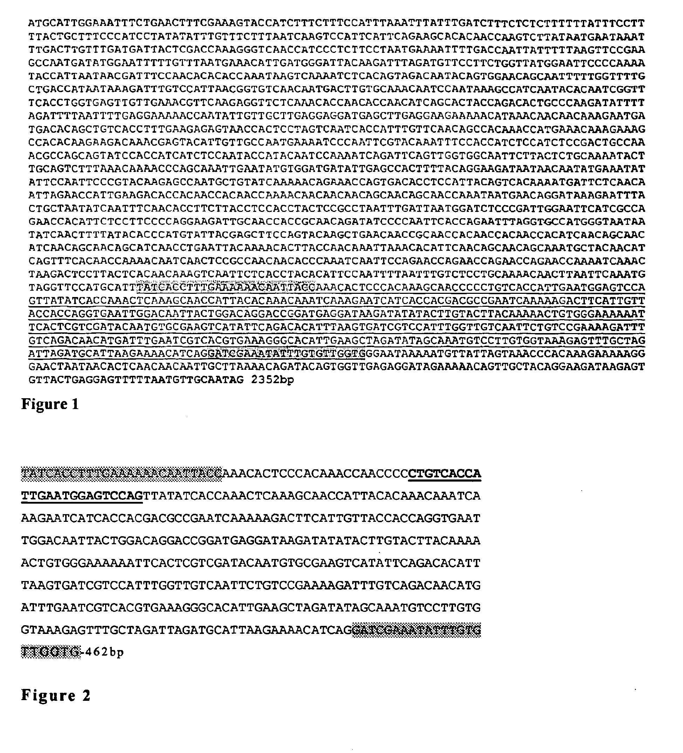

**Figure 1****:** Primers binding sites in (grey highlights) Ace2 gene in *Candida albicans* (XM_707274.1). The amplified region of interest is underlined (Position 1736 to 2197). Product size= 462 base pairs
**Figure 2****:** Location of *C*. *albicans* probe P1-CanAce2 binding site (underlined and bolded in the amplified fragment of Ace2 gene. PCR primers CanAce2-F/CanAce2-R are highlighted.

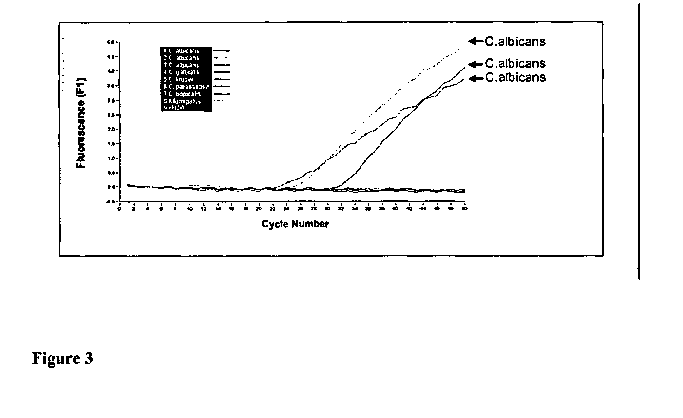

**Figure 3****:** Real-time PCR assay for *C*. *albicans* based on the Ace2 gene with TaqMan probe P1-CanAce2. Specificity of the assay was tested using a panel of DNA from four other Candida species and *Aspergillus fumigatus.* The three *C. albicans* strains tested were detected in the real-time PCR and there was no cross-reaction with DNA for other species.

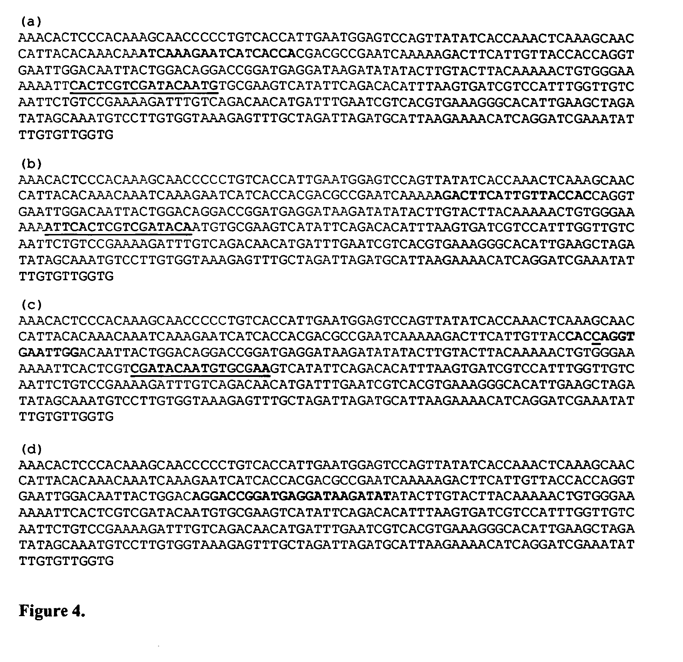

**Figure** 4: Location of indicated *C*. *albicans* primers and probes. Figure 4(a) primer ACF1 is marked in bold, while primer ACR1 is marked in bold underline; Figure 4(b) primer ACF2 is marked in bold and primer ACR2 is marked in bold underline; Figure 4(c) primer ACF3 is marked in bold and primer ACR3 is marked in bold underline; and Figure 4(d) probe is marked in bold, ACALB1 on - strand, ACALB2 in + strand.

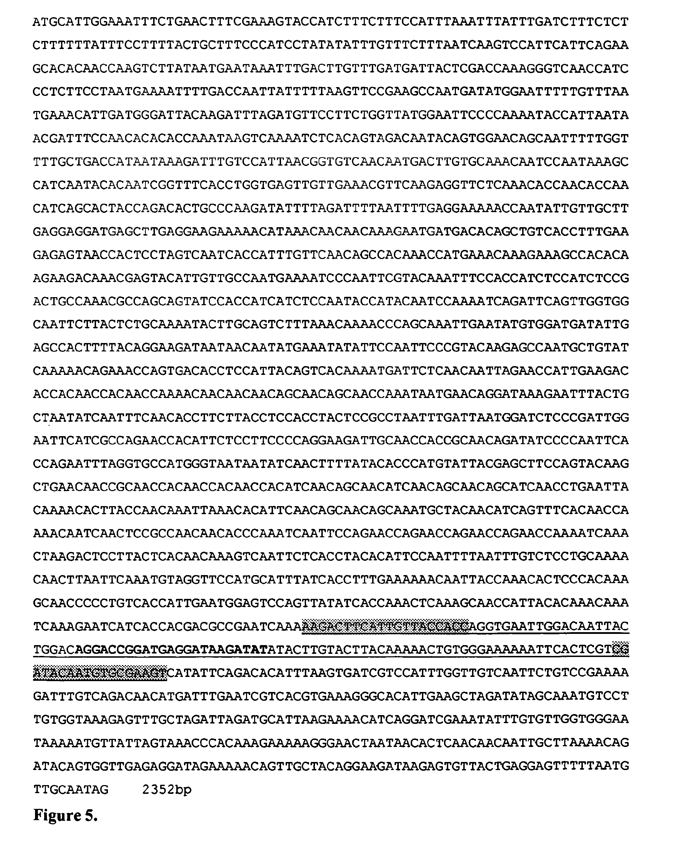

**Figure 5****:** Primer (ACF2b/ACR3b) binding sites (grey highlights) and ACALB1 probe (bold text) in Ace2 of *Candida albicans* (XM_707274.1). The amplified region of interest is underlined. (Position of the region of interest: 1879-2004).

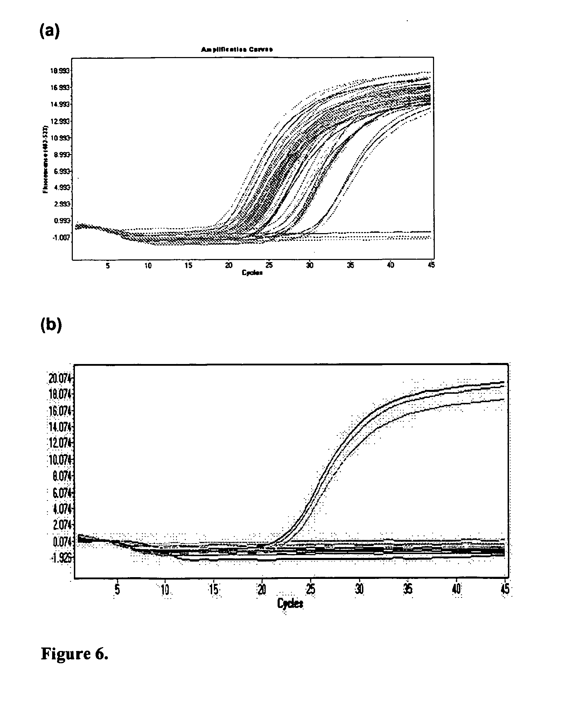

**Figure 6****:** Inclusivity panel of *C*. *albicans* strains. Amplification plot from Real-time PCR assay for *C. albicans* based on the Ace2 gene with TaqMan probe ACALB1 and primer set ACF2b/ACR3b. Specificity of the assay was tested using a panel of DNA from 20 *C*. *albicans* strains and 84 strains covering 19 spp. of clinically relevant and related *Candida.* (a) The 20 *C*. *albicans* strains tested were detected. (b) No cross-reaction was seen with DNA from 84 strains covering 19 other *Candida* species. Signal obtained only from (+) controls.

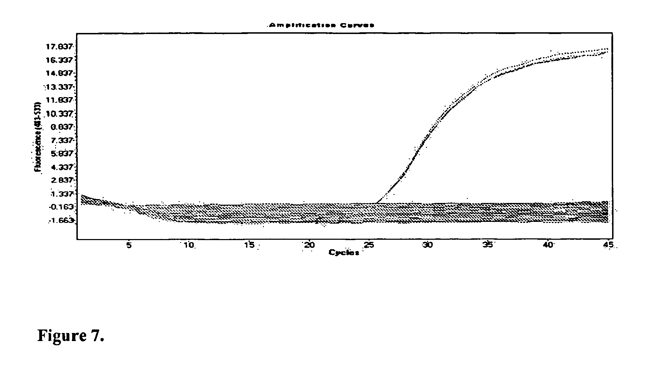

**Figure 7****:** Exclusivity vaginitis/ vaginosis panel. Amplification plot from Real-time PCR assay for *C*. *albicans* based on the Ace2 gene with TaqMan probe ACALB1 and primer set ACF2b/ACR3b. A signal was obtained only for (+) control (*C. albicans*) samples. No signal was obtained for the organisms in the exclusivity panel.

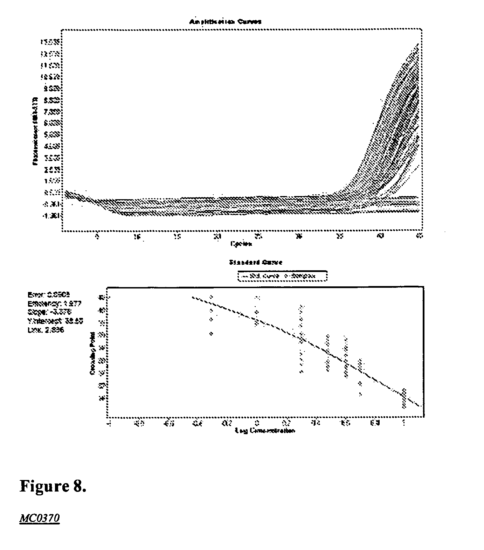

**Figure 8****:** Amplification plot from Real-time PCR assay for *C*. *albicans* based on the Ace2 gene with TaqMan probe ACALB1. Sensitivity of the assay was tested using various inputs of template DNA from *C. albicans.* The LOD of the assay was found to be ∼1 cell equivalent.

### Detailed Description of the Drawings

### EXAMPLE 1

### Materials and Methods

### Cell Culture

*Candida* species were cultured in Sabouraud broth (4% wt/vol glucose, 1% wt/vol peptone, 1.5 % agar) for 48 hours at 37°C in a shaking incubator.

### DNA Extraction

Cells from *Candida* spp. were pre-treated with lyticase or zymolase enzymes prior to DNA isolation. DNA was isolated from *Candida* spp. using either the MagNA Pure System (Roche Molecular Systems) in combination with the MagNA pure Yeast and Bacterial isolation kit III according to the manufacturers protocol, or the Qiagen DNeasy Plant Mini kit (silica-based DNA purification in spin column format).

### DNA sequencing of Ace2 gene regions in Candida species

The publicly available sequences of the Ace2 genes of *Candida* species were acquired from the NCBI database and aligned using ClustalW. The PCR Primer set, namely CanAce2-F/CanAce2-R (Figure 1, Table 1) was designed and used to amplify the Ace2 gene region in *Candida* spp. equivalent to base pair position 1736 to base pair position 2197 in *C*. *albicans* with primer set CanAce2-F/CanAce2-R. The Ace2 gene regions were amplified in *C*. *albicans, C.tropicalis* and *C*. *dubliniensis* by conventional PCR on the iCycler BioRad PCR machine or the PTC200 Peltier thermocycler (MJ Research) using the reagents outlined in Table 2 and the thermocycling conditions described in Table 3.

The PCR reaction products were purified with Roche High Pure PCR Product Purification kit or with the ExoSAP-IT kit (USB) according to the manufacturers' protocol and subsequently sent for sequencing to Sequiserve, Germany using the forward amplification primer CanAce2-F. DNA sequence information was generated for three Candida species. (*C*. *albicans, C. tropicalis and C. dubliniensis*).

**Table 1: PCR primers designed to amplify the Ace2 gene regions in Candida spp.**

| Primer Name | Primer Sequence |
|---|---|
| CanAce2-F | TATCACCTTTGAAAAAACAATTACC |
| CanAce2-R | CACCAACACAAATATTTCGATC |

**Table 2: Conventional PCR reagents used to amplify the Ace2 gene regions in Candida spp.**

| **PCR Reaction Mix** | SAMPLE |
|---|---|
| | x 1 |
| 10 x Buffer(100 mM Tris HCl, 15 mM MgCl₂, 500 mM KCl pH 8.3) | 5 µl |
| dNTP's Mix, Roche (10mM dNTP) | 1 µl |
| Primer Forward CanAce2-F (10µM) | 1 µl |
| Primer Reverse CanAce2-R (10µM) | 1 µl |
| Polymerase TaqPol, Roche 1U/µl | 1 µl |
| H₂O Amgen/Accugene | 36-39 µl |
| Genomic DNA Template | 2-5 µl |
| TOTAL VOLUME | 50 µl |

**Table 3: Conventional PCR reaction conditions applied to amplify the Ace2 gene regions in Candida spp.**

| **PCR Thermal profile** | | Lid preheating was **ON** | |
|---|---|---|---|
| Step | Temp | Time | |
| 1 | 94°C | 1 min | |
| 2 | 53 or 50°C | 1 min | **X 35** |
| 3 | 72°C | 1 min | |
| 4 | 72°C | 7 min | |
| 5 | 8°C | Hold | |

**Table 4: TaqMan probe for C. albicans based on Ace2 gene region**

| | |
|---|---|
| P1-CanAce2 | CTGTCACCATTGAATGGAGTCCAG |

**Table 5: Real-time PCR reagents**

| **Preparation of PCR Reaction Mix** | SAMPLE |
|---|---|
| LightCycler®FastStartDNA Master HybProbe, Roche Cat. 03 003 248001 | x 1 |
| HybProb mix 10 x conc. (Red cap) | 2 µl |
| MgCl₂ stock solution (Blue cap) (Final conc. in reaction is 3 mM) | 1.6 µl |
| Probe P1-CanAce2 | 2 µl |
| Primer Forward CanAce2-F | 1 µl |
| Primer Reverse CanAce2-R | 1 µl |
| H₂O PCR-grade | 10.4 µl |
| Template | 2 µl |
| TOTAL VOLUME | 20 µl |

**Table 6: Real-time PCR thermocycling conditions**

| **PCR Thermal profile** | | | | |
|---|---|---|---|---|
| **Cycle** | **Step** | **Temp** | **Time** | |
| Activation | 1 | 95°C | 10 min | |
| Amplification | 1 | 95°C | 10 sec | **X 50** |
| | 2 | 62-65°C | 20 sec | |
| | 3 | 70°C | 10 sec | |
| Cooling | 1 | 40°C | Hold | |

### RESULTS

### Primer and Probe Design

The sequence information available for the Ace2 gene in *Candida* spp. was aligned with the newly generated sequence information for the Ace2 gene in *Candida* spp. and analysed using bioinformatics tools. Species-specific probes were designed based on the compiled Ace2 sequence information for *Candida albicans* (Table 4). Figures 2 shows the relative positions of the PCR primers and TaqMan DNA probe for the amplification and detection of *C*. *albicans.* The specificity of the TaqMan probes for the identification of *C*. *albicans* was demonstrated in a real-time PCR assay on the LightCycler using the reagents and thermocycling conditions outlined in Tables 5. For the *C*. *albicans* assay based on the Ace2 gene, PCR primers CanAce2-F/CanAce2-R were combined with TaqMan probe, P1-CanAce2. The specificity of the assay for the detection of *C*. *albicans* was confirmed by including DNA from a range of closely related Candida species and *A. fumigatus* species in the *C*. *albicans* real-time PCR assay. The assay detected *C*. *albicans* but did not detect or cross-react with DNA from any other Candida species tested or with *A. fumigatus* DNA. Figure 3 shows the *C*. *albicans* real-time PCR assay and the specificity of the assay for *C*. *albicans.*

### EXAMPLE 2

Primers and probes for specific detection and identification were designed following *in silico* analysis of generated sequences. Three forward and three reverse primers were generated and two probes were designed as follows. Figures 4(a) to 4(d) discloses the location of these sequences in the Ace2 subsequence.
ACF1: SEQ ID NO:20: ATCAAAGAATCATCACCA
ACF2: SEQ ID NO:21: AGACTTCATTGTTACCAC
ACF3: SEQ ID NO:24: CACCAGGTGAATTGG
ACR1: SEQ ID NO:25: CATTGTATCGACGAGTG
ACR2: SEQ ID NO:26: TGTATCGACGAGTGAAT
ACR3: SEQ ID NO:27: TTCGCACATTGTATCG
ACALB1: SEQ ID NO:30: 6FAM-ATATCTTATCCTCATCCGGTCCT--BHQ1
ACALB2: SEQ ID NO:31: 6FAM-AGGACCGGATGAGGATAAGATAT--BHQ1

The primer sets were evaluated using the following assay conditions: UNG treatment: 50°C 2min followed by 95°C 1min. The amplification included 50 cycles, 95°C 10sec, 60°C 30 sec, followed by a 2 min cooling at 40°C.

Based on initial assay performance (e.g. fluorescence and efficiency), primer set ACF3/ ACR3 was chosen for further evaluation using ACALB1 probe. Initial inclusivity and exclusivity experiments were performed to evaluate the potential of the assay using the chosen assay oligonucleotides. Specificity of the assay was tested using a panel of DNA from 14 *C*. *albicans* strains and 23 strains representing 19 other *Candida* species. All 14 *C*. *albicans* strains tested were detected. No cross-reaction was seen with DNA from 19 the other *Candida* species, *i.e.,* signal was obtained only from the positive *C*. *albicans* control.

The Ace2 assay was shown to be specific to *C*. *albicans,* and initial performance was good under conditions tested.

Next, eight different combinations of primers were tested in order to reduce cycling times. The following conditions were tested: UNG treatment for 2min at 50°C, 1min at 95°C, followed by amplification of 50 cycles, 95°C 5sec, 60°C 10sec, followed by a cooling step of 2min at 40°C.

The combination of primer set ACF2/ACR3 and the ACALB1 probe performed the best under these conditions. The LOD of the assay is ∼1-10 cell equivalents. This primer combination demonstrated higher fluorescence at lower template inputs, and 3 out of 3 one-cell equivalents were detected with earlier Cp values. Slight modifications were made to the primer sequences in order to further shorten cycle times, while retaining sensitivity and specificity (Table.7).

**Table 7: Modifications to primer sequences**

| Primer name | SEQ ID NO | Tm |
|---|---|---|
| ACF2 | 21 | 53°C |
| ACF2b | 22 | 57.1°C |
| ACF2c | 23 | 59.5°C |
| ACR3 | 27 | 52.7°C |
| ACR3b | 28 | 56.8°C |
| ACR3c | 29 | 60.2°C |

Further reduced cycling conditions were tested, *i.e.,* amplification step of 95°C for 1 second and 60°C for 10 seconds. 10-fold dilutions of *C*. *albicans* DNA were prepared and inputs of 1x10⁵ to 0.1 cell equivalents were used as template. The combination of ACF2b and ACR3b primers were selected for inclusivity and exclusivity testing due to higher fluorescence at lower template inputs and 3 out of 3 one-cell equivalents were detected with earlier Cp values than other primer sets, as indicated in Table 8.

**Table 8: CP values obtained for different Tm primer sets.**

| **Average CP values for samples** | | | | | | |
|---|---|---|---|---|---|---|
| **Template input** | **ACF2/ACR3** | | **ACF2b/ACR3b** | | **ACF2c/ACR3c** | |
| 100000 | (3/3) | 21.92 | (3/3) | 22.08 | (3/3) | 22.52 |
| 1000 | (3/3) | 25.55 | (3/3) | 25.66 | (3/3) | 25.95 |
| 1000 | (3/3) | 29.05 | (3/3) | 29.19 | (3/3) | 29.60 |
| 109 | (3/3) | 32.56 | (3/3) | 32.87 | (3/3) | 33.01 |
| 10 | (3/3) | 35.66 | (3/3 | 36.05 | (3/3) | 36.59 |
| 1 | (2/3) | 40 | (3/3) | 39.34 | (3/3) | 40 |
| 0.1 | (1/3) | 40 | | 0 | | 0 |

Twenty strains of *Candida albicans* were selected to demonstrate inclusivity of detection with this primer set (see Table 9, Figure 5 and Figure 6). Real-time PCR assay for *C*. *albicans* was carried out as described with TaqMan probe ACALB1 and primer set ACF2b/ACR3b. All twenty *C*. *albicans* strains tested were detected, and no signal obtained from (-) controls (see Figure 6(a)).

**Table 9: C. albicans strains used in inclusivity experiments**

| No. | Strain number | Strain name | Source | Additional Information | Country | Detector |
|---|---|---|---|---|---|---|
| 1 | 3156 | *C.albicans* | PHLS/NCPF | 1965 Serotype B | UK | ✔ |
| 2 | 3345 | *C.albicans* | PHLS | Arm abscess | UK | ✔ |
| 3 | 3328 | *C.albicans* | PHLS | Renal transplant patient | UK | ✔ |
| 4 | 178 | *C.albicans* | UCHG | Clinical isolate | Galway Ireland | ✔ |
| 5 | 180 | *C.albicans* | UCHG | Clinical isolate | Galway Ireland | ✔ |
| 6 | 320 | *C.albicans* | UCHG | Clinical isolate | Galway Ireland | ✔ |
| 7 | 369 | *C.albicans* | UCHG | Clinical isolate | Galway Ireland | ✔ |
| 6 | 765 | *C.albicans* | UCHG | Clinical isolate | Galway Ireland | ✔ |
| 9 | 562 | *C.albicans* | CBS | patient with interdigital mycosi | Urugauy | ✔ |
| 10 | 15640 | *C.albicans* | IHEM | Blood, tuberculosis | Belgium | ✔ |
| 11 | 9559 | *C.albicans* | IHEM | Blood | Iowa USA | ✔ |
| 12 | 16733 | *C.albicans* | IHEM | Blood, Candidemia AIDS | Belgium | ✔ |
| 13 | 1893 | *C.albicans* | CBS | Soil | New Zealand | ✔ |
| 14 | 1899 | *C.albicans* | CBS | man, infected skin of child | Republic of Congo | ✔ |
| 15 | 1912 | *C.albicans* | CBS | man, sputum, asthma patient | Norway | ✔ |
| 16 | 2688 | *C.albicans* | CBS | infection of urino-genital tract | Switzerland, Bern | ✔ |
| 17 | 2701 | *C.albicans* | CBS | man, pus from lung, dead mal | Italy, Pavia | ✔ |
| 18 | 8642 | *C.albicans* | CBS | sputum, from 19-year-old man | Germany, Dresden | ✔ |
| 19 | 2738 | *C.albicans* | CBS | man, mouth of child | Portugal, Oporto | ✔ |
| 20 | 2700 | *C.albicans* | CBS | macroglossia mycotica | Brazil | ✔ |

In order to demonstrate exclusivity of the assay, the Ace2 assay was tested for performance against 84 strains covering 19 spp. of clinically relevant and related *Candida* (see Table 10 below). In order to ensure that all DNA tested is PCR amplifiable, the *Candida* DNA samples were amplified with universal fungal primers U1 and U2. All DNA tested was shown to be PCR amplifiable. A template input of 10⁵ CE/rxn was used, which is higher than normally tested. A higher input was tested because the organisms tested are related *Candida* and Ace2 sequence information was not confirmed for 17 species prior to testing. Each input was tested in triplicate. No cross-reactions were observed, demonstrating the specificity of the Ace2 assay for *C*. *albicans* detection (see Figure 6(b)).

**Table 10: Candida Specificity panel - Clinically relevant and related Candida spp.**

| | ***C. albicans* Ace2 specificity panel - Tested in triplicate** | | | | |
|---|---|---|---|---|---|
| 1 | 3605 | *C.glabrata* | PHLS NCPF | Diabetes | UK |
| 2 | 8018 | *C.g*/*abrata* | PHLS NCPF | Blood | UK |
| 3 | 3863 | *C.glabrata* | PHLS NCPF | Blood | UK |
| 4 | 90876 | *C.glabrata* | ATCC | Blood | Germany |
| 5 | 3959 | *C.glabrata* | IHEM | Blood | Belgium, Leuven |
| 6 | 9556 | *C.glabrata* | IHEM | Blood | Brussels, Belgium |
| 7 | 138 | *C.glabrata* | CBS | | ? |
| 8 | 860 | *C.glabrata* | CBS | man, urine, of diabe | Netherlands |
| 9 | 861 | *C.glabrata* | CBS | mouth | Netherlands, Groningen |
| 10 | 7307 | *C.glabrata* | CBS | sputum, of 63-year- | New Zealand, Masterton |
| | | | | | |
| 1 | 3873 | *C.tropicalis* | PLHS NCPF | Blood, neutropenic | UK |
| 2 | 3870 | *C.tropicalis* | PLHS NCPF | Blood | UK |
| 3 | 4225 | *C.tropicalis* | IHEM | Blood | Brussels, Belgium |
| 4 | 5557 | *C.tropicalis* | IHEM | Blood | Grenoble, France |
| 5 | 94 Type | *C.tropicalis* | CBS | | ? |
| 6 | 2313 | *C.tropicalis* | CBS | superficial interdigit | Egypt, Cairo |
| 7 | 5701 | *C.tropicalis* | CBS | infected hand | Hungary |
| 8 | 6361 | *C.tropicalis* | CBS | blood, of 53-year-olg | USA |
| 9 | 8072 | *C.tropicalis* | CBS | man, blood, at auto | USA, New York |
| 10 | 10256 | *C.tropicalis* | CBS | blood sample | France, Paris |
| | | | | | |
| 1 | 5579 | *C.krusei* | IHEM | Blood | France |
| 2 | 9560 | *C.krusei* | IHEM | Spumum, bronchon | Sri Lanka |
| 3 | 2052 | *C.krusei* | CBS | sputum from case | Italy, Milano |
| 4 | 2046 | *C.krusei* | CBS | pus from infected fir | Argentina, Buenos Aires |
| 5 | Type 573 | *C.krusei* | CBS | Sputum | Sri Lanka |
| 6 | 3165 | *C.krusei* | PHLS NCPF | Sputum | UK |
| | 7 | *C.krusei* | PHLS NCPF | Human blood | UK |
| 8 | 3847 | *C.krusei* | PHLS NCPF | Abdominal fistula | UK |
| 9 | 3433 | *C.krusei* | DSMZ | fruit juice or berries | n/a |
| 10 | 70075 | *C.krusei* | DSMZ | human faeces | n/a |
| | | | | | |
| 1 | 96137 | *C.parapsilolsis* | ATCC | Leg wound | Texas |
| 2 | 1716 | *C*.*parapsilosis* | IHEM | Human nail, onychc | Gosselies, Belgium |
| 3 | 604 | *C.parapsilosis* | CBS | | Puerto Rico |
| 4 | 2194 | *C.parapsilosis* | CBS | | Austria |
| 5 | 2196 | *C.parapsilosis* | CBS | | Dominican Republic |
| 6 | 2197 | *C.parapsilosis* | CBS | bladder | Denmark, Copenhagen |
| 7 | 4024 | *C.parapsilosis* | IHEM | human ear canal | Belgium |
| 8 | 2916 | *C.parapsilosis group III metapsilosis* | CBS | sputum | Norway |
| 9 | 96143 | *C.parapsilosis group III metapsilosis* | ATCC | unknown | California |
| 10 | 96141 | *C.parapsilosis gropu II orthopsilosis* | ATCC | Blood | Texas |
| 1 | *3949 Type | *C.dubliniensis* | PHLS NCPF | cavity HIV + | Ireland |
| | *14280 Type | *C.dubliniensis* | IHEM | | **Same strain as 3949 |
| 2 | 16625 | *C.dubliniensis* | IHEM | human mouth | Besançon, France |
| 3 | 16971 | *C.dubliniensis* | IHEM | human sputum | Brussels, Belgium |
| 4 | 7988 | *C.dubliniensis* | CBS | man, oral cavity, HI | Australia, Victoria, Melbourne |
| 5 | 7987 | *C.dubliniensis* | CBS | oral cavity of | Ireland, Dublin |
| 6 | 8500 | *C.dubliniensis* | CBS | blood, of | Netherlands, Nijmegen |
| 7 | 8501 | *C.dubliniensis* | CBS | child, with neutrope | Netherlands, Nijmegen |
| | | | | | |
| 1 | 8013 | *C.lusitame* | PHLS NCPF | Sputum, hydroneph | UK |
| 2 | 19472 | *C.lusitanie* | IHEM | citrus fruit | Isreal |
| 3 | 7270 | *C.lusitanie* | CBS | sputum, from pneur | New Zealand |
| 4 | 10294 | *C.lusitanie* | IHEM | | Maryland USA |
| 5 | 4600 | *C.lusitanie* | IHEM | human blood candic | Italy, Milano |
| | | | | | |
| 1 | 8167 | *C.guilhermondii* | PHLS NCPF | Blood, patient with | UK |
| 2 | 2672 | *C.guilhormondii* | CBS | case of cystitis, man | |
| 3 | 6021 | *C.guilliermondii* | CBS | soil | Japan, Niigata Province |
| 4 | 2682 | *C.guilliermondii* | CBS | semen of bull | UK |
| | | | | | |
| 1 | 2070 | *C.lipolytica* | CBS | corneal lesion | Italy, Siena |
| 2 | 2071 | *C.lipolytica* | CBS | man, finger nail, 49 | Austria |
| 3 | 8218 | *C.lipolytica* | DSMZ | Diesel fuel storage | USA |
| | | | | | |
| 1 | 5312 | *C.rugosa* | CBS | sake starter yeast | Japan |
| 2 | 613 | *C*. *rugosa* | CBS | faeces of Man | ? |
| 3 | 1948 | *C. rugosa* | CBS | sputum | Norway |
| 4 | 6703 | *C*. *rugosa* | IHEM | human urine | Belgium, Brussels |
| | | | | | |
| 1 | 1922 | *C. norvegensis* | CBS | sputum, of patient | Norway |
| 2 | 2144 | *C. norvegensis* | CBS | sputum | Netherlands, Gouda |
| 3 | 2145 | *C*. *norvegensis* | CBS | abdominal infiltrate | Netherlands, Dordrecht |
| | | | | | |
| 1 | 5295 | *Stephanoascus ciferrii* | CBS | piq, with infected th | Netherlands |
| 2 | 70749 | *Stephanoascus ciferrii* | DSMZ | dried yeast | N/A |
| | | | | | |
| 1 | 70040 | *C. catenulata* | DSMZ | ? | Frankfurt |
| 2 | 70136 | *C. catenulata* | DSMZ | hyperkeratinic foot | N/a |
| | | | | | |
| 1 | 3428 | *C. famata* | DSMZ | spoilt sake | nla aka Debaryomyces hansenii |
| 2 | 70590 | *C. famata* | DSMZ | harzer cheese | n/a aka Debaryomyces hansenii |
| | | | | | |
| 1 | 7802 | *C. haemulori* | CBS | ulcer on toe | USA |
| 2 | 70624 | *C. haemuloni* | DSMZ | intestinal tract of *Ha* | Florida |
| | | | | | |
| 1 | 3898 | *C.keyfr* | PHLS NCPF | Blood, neutropenic | UK |
| 2 | 70073 | *C.keyfr* | DSMZ | | aka Kluveromyces marxianus |
| | | | | | |
| 1 | 70336 | *C. pulcherrima* | DSMZ | Red dates | Egypt |
| 2 | 70879 | *C. pulcherrima* | DSMZ | flower of *Trifolium p* | n/a |
| | | | | | |
| 1 | 70163 | *C. utilis* | DSMZ | fodder yeast from | |
| 2 | 70167 | *C. utilis* | DSMZ | protein from | n/a |
| | | | | | |
| 1 | 4024 | *C. viswanathii* | CBS | cerebro-spinal fluid, | India |
| 2 | 5362 | *C. viswanathii* | CBS | sputum | India |
| | | | | | |
| 1 | 2000 | *C. zeylanoides* | CBS | sputum, of bronchit | Norway |
| 2 | 70185 | *C. zeylanoides* | DSMZ | Butter | Germany |

Exclusivity was further demonstrated using a vaginitis/vaginosis panel with 45 species of clinically relevant organisms in triplicate (see Table 11 below) using the shorter cycle conditions. A template input of ∼10⁴ CE/rxn was used. No cross-reactions were observed, further demonstrating the specificity of the Ace2 assay (see Figure 7).

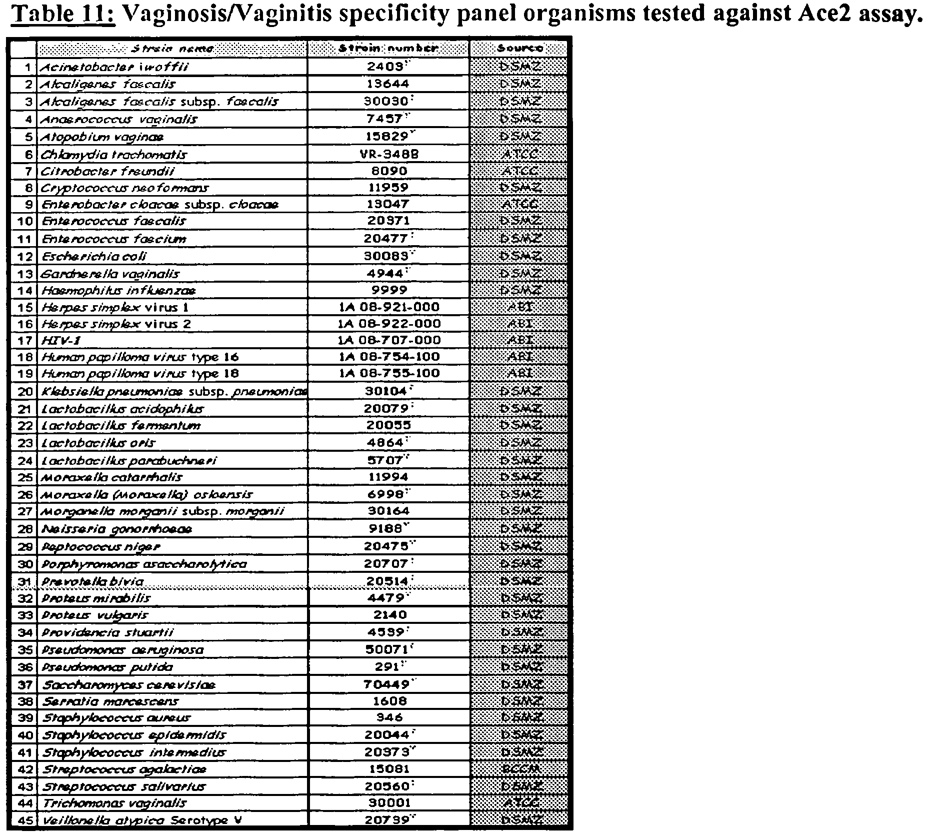

Probit analysis was performed in order to statistically determine the LOD (limit of detection) of the Ace2 assay using ACF2b/ ACR3b/ ACALB1 with shorter cycle times. The LOD of the Ace2 assay was determined by testing 8 template inputs and 12 replicates. *C. albicans* strain CBS 562 was selected for LOD determination. The results are shown below in Table 12 and Figure 8. An LOD of 0.839, with a probability of greater than or equal to 95% was found by Probit analysis.

**Table 12: Input levels (cell equivalents) and results of Probit analysis**

| Number of replicates detected | Input level | Number of replicates tested |
|---|---|---|
| 12 | 10 | 12 |
| 12 | 5 | 12 |
| 12 | 4 | 12 |
| 12 | 3 | 12 |
| 12 | 2 | 12 |
| 11 | 1 | 12 |
| 11 | 0.5 | 12 |
| 1 | 0.1 | 12 |

### Discussion

The number of yeast and fungal infections among immuno-compromised patients is escalating. Contributing to this increase is the growing resistance of many yeast and fungal species to antifungal drugs. There is, therefore, a need to develop a fast, accurate diagnostic method to enable early diagnosis of yeast and fungal species. Early diagnosis will enable the selection of a specific narrow spectrum antibiotic or antifungal to treat the infection. The current invention provides for sequences and/or diagnostic assays to detect and identify one or more yeast and fungal species. The current inventors have exploited the sequence of the Ace2 gene in *Candida* species to design primers and probes specific for regions of this gene. Ace2 is an ideal candidate for the design of primers and probes directed towards the detection of yeast and fungal species-specific targets and for the detection of genus specific diagnostic targets respectively. The current invention allows the detection of yeast and fungal species but also allows distinction between *Candida* species.

### Summary

Using newly generated and publicly available sequences, the inventors have designed new primers and probes that are surprisingly specific to *Candida* Ace2 polynucleotide sequences under short cycle times identified here. Such primers and probes not only allow the detection of yeast and fungal species but also allow surprisingly effective distinction between *Candida* species and specific detection of *C. albicans,* even under short cycle times. The current invention further provides for primers and probes that allow excellent discrimination between different Candida species.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination.

In so far as any sequence disclosed herein differs from its counterpart in the attached sequence listing in PatentIn3.3 software, the sequences within this body of text are to be considered as the correct version.

### SEQ IDs

N or x= any nucleotide; w=a/t, m=a/c, r=a/g, k=g/t, s=c/g, y=c/t, h=a/t/c, v=a/g/c, d=a/g/t, b=g/t/c. In some cases, specific degeneracy options are indicated in parenthesis: e.g.: (a/g) is either A or G.
**SEQ ID NO 1:** CanAce2-F
   TATCACCTTTGAAAAAACAATTACC
**SEQ ID NO 2:** CanAce2-R
   CACCAACACAAATATTTCGATC
**SEQ ID NO 3:** P1-CanAce2
   CTGTCACCATTGAATGGAGTCCAG
**SEQ ID NO 4:**
   >CA3165-Ace2/ACE2F *C*. *albicans* 
AAACACACCAACGAAGCAACCCCCTGTCACCATTGAATGGAGTCCAGTTATATCACCAA
ACTCAAAACAACCATTACACAAACAAATCAAAGAATCATCACCACGACGCCGAATCAAA
AAGACTTCATTGTTACCACCAGGTCAATTGGACAATTACTGGACAGGACCGGATGAGGA
TAAGATATATACTTGTACTTACAAAAACTGTGGAAAAAATTCACTCGTCGATACAATG
TGCGAAGTCATATTCAGACACATTTAAGTGATCGTCCATTTGGTTGTCAATTCTGTCCG
AAPAGATTTGTCAGACAACATGATTTGAATCGTCACGTGAAAGGGCACATTGAAGCTAG
ATATAGCAAATGTCCTTGTGGTAAAGAGTTTGCTAGATTAGATGCATTAAGAAAACATC
AGGATCGAAATATTTGTGTTGGTG
**SEQ ID NO 5:**
   >CA765-Ace2/ACE2F *C*. *albicans* 
AAACACTCCCACAAAGCAACCCCCTGTCACCATTGAATGGAGTCCAGTTATATCACCA
AACTCAAAGCAACCATTACACAAACAAATCAAAGAATCATCACCACGACGCCGAATCA
AAAAGACTTCATTGTTACCACCAGGTGAATTGGACAATTACTGGACAGGACCGGATGA
GGATAAGATATATACTTGTACTTACAAAAACTGTGGAAAAAATTCACTCGTCGATAC
AATGTGCGAAGTCATATTCAGACACATTTAAGTGATCGTCCATTTGGTTGTCAATTCT
GTCCGAAAAGATTTGTCAGACAACATGATTTGAATCGTCACGTGAAAGGCACATTGA
AGCTAGATATAGCAAATGTCCTTGTGGTAAAGAGTTTGCTAGATTAGATGCATTAAGA
AAACATCAGGATCGAAATATTTGTGTTGGTG
**SEQ ID NO 6:**
   >CA562-Ace2/ACE2F *C*. *albicans* 
AAACACT000ACAAAGCAACCCCCTGTCACCATTGAATGGAGTCCAGTTATATCACCAA
ACTCAAAGCAACCATTACACAAACAJATCAAAGAATCATCACCACGACGCCGAATCAAA
AAGACTTCATTGTTACCACCAGGTGAATTGGACAATTACTGGACAGGACCGGATGAGGA
TAAGATATATACTTGTACTTACAAAAACTGTGGCAAAAAATTCACTCGTCGATACAATG
TGCGAAGTCATATTCAGACACATTTAAGTGATCGTCCATTTGGTTGTCAATTCTGTCCG
AAPAGATTTGTCAGACAACATGATTTGAATCGTCACGTGAAAGGGCACATTGAAGCTAG
ATATAGCAAATGTCCTTGTGGTAAAGAGTTTGCTAGATTAGATGCTTTGAGAAAGCACC
AGGATCGAAATATTTGTGTTGGTG
**SEQ ID NO 7:**
   >CA2700-Ace2/ACE2F *C*. *albicans* 
AAACACTCCCACAAAGCAACCCCCTGTCACCATTGAATGGAGTCCAGTTATATCACCAA
ACTCAAAGCAACCATTACACAAACAAATCAAAGAATCATCACCACGACGCCGAATCAAA
AAGACTTCATTGTTACCACCAGGTGAATTCGACAATTACTGGACAGGACCGGATGAGGA
TAAGATATATACTTGTACTTACAAAAACTGTGGAAAAAATTCACTCGTCGATACAATG
TGCGAAGTCATATTCAGACACATTTAAGTGATCGTCCATTTGGTTGTCAATTCTGTCCG
AAPAGATTTGTCAGACAACATGATTTGAATCGTCACGTGAAAGGCACATTGAAGCTAG
ATATAGCAAATGTCCTTGTGGTAAAGAGTTTGCTAGATTAGATGCATTAAGAAAACATC
AGGATCGAAATATTTGTGTTGGTG
**SEQ ID NO 8:**
   >CD3494 /ACE2F *C*. *dubliniensis* 
AAACACTCCCACAAAGCAACCCCCTGTCACCATTGAATGGAGTCCAGTTATATCACCAA
ACTCAAAGCAACCATTACACAAACAAATCAAAGAATCGTCACCACGACGCCGAATCAAA
AAGACCTCATTGTTGCCACCAGGTGAATTGGATAATTACTGGACAGGACCGGATGAGGA
TAAGATATATACTTGTACTTACAAAAACTGTGGAAAAAATTCACTCGTCGATATAATG
TGCGAAGTCATATTCAAACACATTTAAGTGATCGTCCATTTGGTTGTCAGTTCTGTCCC
AAAAGATTTGTTAGACAACATGATTTGAATCGTCACGTGAAAGGACACATTGAAGCCAG
ATATAGTAAATGTCCTTGTGGTAAAGAGTTTGCTAGATTAGATGCTTTGAGAAAGCACC
AGGATCGAAATATTTGTGTTGGTG
**SEQ ID NO 9:**
   >CD7987 /ACE2F *C. dubliniensis* 
AAACACACCAACGAAGCAACCCCCTGTCACCATTGAATGGAGTCCAGTTATATCACCAA
ACTCAAAACAACCATTACACAAACAAATCAAAGAATCGTCACCACGACGCCGAATCAAA
AAGACCTCATTGTTGCCACCAGCTGAATTGGATAACTATTGGACAGGACCAGATAAAGA
CAAAATATATACCTGTACCTACAAAAACTGTGGAAAAAATTCACTCGTCGATATAATG
TGCGAAGTCATATTCAAACACATTTAAGTGATCGTCCATTTGGTTGTCAGTTCTGTCCC
AAAAGATTTGTTAGACAACATGATTTGAATCGTCACGTGAAAGGACACATTGAAGCCAG
ATATAGTAAATGTCCTTGTGGTAAAGAGTTTGCTAGATTAGATGCTTTGAGAAAGCACC
AGGATCGAAATATTTGTGTTGGTG
**SEQ ID NO 10:**
   >CD8501S23 /ACE2F *C*. *dubliniensis* 
AACACACCAACGAAGCAACCCCCTGTCACCATTGAATGGAGTCCAGTTATATCACCAAA
CTCAAAACAACCATTACACAAACAAATCAAAGAATCGTCACCACGACGCCGAATCAAAA
AGACCTCATTGTTGCCACCAGGTGAATTGGATAATTACTGGACAGGACCGGATGAGGAT
AAGATATATACTTGTACTTACAAAAACTGTGGAAAAAATTCACTCGTCGATATAATGT
GCGAAGTCATATTCAAACACATTTAAGTGATCGTCCATTTGGTTGTCAGTTCTGTCCCA
AAAGATTTGTTAGACAACATGATTTGAATCGTCACGTGAAAGGACACATTGAAGCCAGA
TATAGTAAATGTCCTTGTGGTAAAGAGTTTGCTAGATTAGATGCTTTGAGAAAGCACCA
GGATCGAAATATTTGTGTTGGTG
**SEQ ID NO 11:**
   >94-Ace2/ACE2F *C*. *tropicalis* 
TATCACCTTTGAAAPCCAATTACCAAGTACACCAACTAAACAACAAAATTCCCAAGTTA
CTATTGAATGGAGCCCTGTTATTTCACCAAATTCCAAAGCATCATTATCTAAACAACTC
AAAGAATCATCATTACAAACGTCGCCACGTAGAAGAATTAAAAAGACTTCATTATTGCC
ACCTGGAGAACTTGATAATTATTGGATTGGACCCGATGAAAATAAAATTTATACATGTA
CTTATAAAAACTGTTTTAAAAAATTTACAAGAAGATATAATGTTAGAAGTCATATTCAA
ACTCATTTTGTCCTAAAAGATTTGTTCGACAACATGATTTAAATCGTCATGTTAAAGGA
CATATTGAAGCAAGATATAGTAAATGGACATATTGAAGCAAGATATAGTAAATGTCCAT
GTGGTAAAGAATTTGCAAGATTAGATGCTTTAAGAAAACATC
AAGATCGAAATATTTGTATTGGTG
**SEQ ID NO 12:**
   >CT8157-Ace2/ACE2F *C*. *tropicalis* 
TACACCAACTAAACAACAAAATTCCCAAGTTACTATTGAATGGAGCCCTGTTATTTCAC
CAAATTCCAAAGCATCATTATCTAAACAACTCAAAGAATCATCATTACAAACGTCGCCA
CGTAGAAGAATTAAAAAGACTTCATTATTGCCACCTGGAGAACTTGATAATTATTGGAT
TGGACCCGATGAAAATAAAATTTATACATGTACTTATAAAAACTGTTTTAAAAAATTTA
CAAGAAGATATAATGTTAGAAGTCATATTCAAACTCATTTAPGTGATAGACCATTTGGA
TGTCAATTTTGTCCTAAAAGATTTGTTCGACAACATGATTTAAATCGTCATGTTAAAGG
ACATATTGAAGCAAGATATAGTAAATGTCCATGTGGTAAAGAATTTGCAAGATTAGATG
CTTTAAGAAAACATCAAGATCGAAATATTTGTGTTGGTG
**SEQ ID NO 13:**
   >CT2311-Ace2/ACE2F *C. tropicalis* 
TACACCAACTAAACAACAAAATTCCCAAGTTACTATTGAATGGAGCCCTGTTATTTCAC
CAAATTCCAAAGCATCATTATCTAAACAACTCAAAGAATCATCATTACAAACGTCGCCA
CGTAGAAGAATTAAAAAGACTTCATTATTGCCACCTGGAGAACTTGATAATTATTGGAT
TGGACCCGATGAAAATAAAATTTATACATGTACTTATAAAAACTGTTTTAAAAAATTTA
CAAGAAGATATAATGTTAGAAGTCATATTCAAACTCATTTAAGTGATAGACCATTTGGA
TGTCAATTTTGTCCTAAAAGATTTGTTCGACAACATGATTTAAATCGTCATGTTAAAGG
ACATATTGAAGCAAGATATAGTAAATGTCCATGTGGTAAAGAATTTGCAAGATTAGATG
CTTTAAGAAAACATCAAGATCGAAATATTTGTGTTGGTG
**SEQ ID NO 14:**
   >CT2424-Ace2/ACE2F *C*. *tropicalis* 
TACACCAACTAAACAACAAAATTCCCAAGTTACTATTGAATGGAGCCCTGTTATTTC
ACCAAATTCCAAGCATCATTATCTAAACAACTCAAAGAATCATCATTACAAACGTCG
CCACGTAGAAGAATTAAAAAGACTTCATTATTGCCACCTGGAGAACTTGATAATTAT
TGGATTGGACCCGATGAAAATAAAATTTATACATGTACTTATAAAAACTGTTTTAA
AAAATTTACAAGAAGATATAATGTTAGAAGTCATATTCAAACTCATTTAAGTGATAG
ACCATTTGGATGTCAATTTTGTCCTAAAAGATTTGTTCGACAACATGATTTAAATCG
TCATGTTAAAGGACATATTGAAGCAAGATATAGTAAATGTCCATGTGGTAAAGAATT
TGCAAGATTAGATGCTTTAAGAAAACATCAAGATCGAAATATTTGTGTTGGTG
**SEQ ID NO 15:**
   >gi1684874911refIXM_707274.1I *Candida albicans* SC5314 putative transcription factor (Ca019.13543), mRNA GENE ID: 3645995 ace2 1 putative zinc finger protein similar to *S. cerevisiae* ACE2 (YLR131C) cell cycle-specific transcriptional regulator of CUP 1 [*Candida albicans* SC5314] 
ATGCATTGGAAATTTCTGAACTTTCGAAAGTACCATCTTTCTTTCCATTTAAATTTA
TTTGATCTTTCTCTCTTTTTTATTTCCTTTTACTGCTTTCCCATCCTATATATTTGT
TTCTTTAATCAAGTCCATTCATTCAGAAGCACACAACCAAGTCTTATAATGAATAAA
TTTGACTTGTTTGATGATTACTCGACCAAAGGGTCAACCATCCCTCTTCCTAATGAA
 
AATTTTGACCAATTATTTTTAAGTTCCGAAGCCAATGATATGGAATTTCTCTTTTTT
ATTTCCTTTTACTGCTTTCCCATCCTATATATTTGTTTCTTTAATCAAGTCCATTCA
TTCACCATTAATAACGATTTCCAACACACACCAAATAAGTCAAAATCTCACAGTAGA
CAATACAGTGGAACAGCAATTTTTGGTTTGCTGACCATAATAAAGATTTGTCCATT
AACGGTGTCAACAATGACTTGTGCAAACAATCCAATAAAGCCATCAATACACAATCG
GTTTCACCTGGTGAGTTGTTGAAACGTTCAAGAGGTTCTCAAACACCAACACCAACA
TCAGCACTACCAGACACTGCCCAAGATATTTTAGATTTTAATTTTGAGGAAAAACCA
ATATTGTTGCTTGAGGAGGATGAGCTTGAGGAAGAAAAACATAAACAACAACAAAGA
ATGATGACACAGCTGTCACCTTTGAAGAGAGTAACCACTCCTAGTCAATCACCATTT
GTTCAACAGCCACAAACCATGAAACAAAGAAAGCCACACAAGAAGACAAACGAGTAC
ATTGTTGCCAATGAAAATCCCAATTCGTACAAATTTCCACCATCTCCATCTCCGACT
GCCAAACGCCAGCAGTATCCACCATCATCTCCAATACCATACAATCCAAAATCAGAT
TCAGTTGGTGGCAATTCTTACTCTGCAAAATACTTGCAGTCTTTAAACAAAACCCAG
CAAATTGAATATGTGGATGATATTGAGCCACTTTTACAGGAAGATAATAACAATATG
AAATATATTCCAATTCCCGTACAAGAGCCAATGCTGTATCAAAAACAGAAACCAGTG
ACACCTCCATTACAGTCACAAAATGATTCTCAACAATTAGAACCATTGAAGACACCA
CAACCACAACCAAAACAACAACAACAGCAACAGCAACCAAATAATGAACAGGATAAA
GAATTTACTGCTAATATCAATTTCAACACCTTCTTACCTCCACCTACTCCGCCTAAT
TTGATTAATGGATCTCCCGATTGGAATTCATCGCCAGAACCACATTCTCCTTCCCCA
GGAAGATTGCAACCACCGCAACAGATATCCCCAATTCACCAGAATTTAGGTGCCATG
GGTAATAATATCAACTTTTATACACCCATGTATTACGAGCTTCCAGTACAAGCTGAA
CAACCGCAACCACAACCACAACCACATCAACAGCAACATCAACAGCAACAGCATCAA
CCTGAATTACAAAACACTTACCAACAAATTAAACACATTCAACAGCAACAGCAAATG
CTACAACATCAGTTTCACAACCAAAACAATCAACTCCGCCAACAACACCCAAATCAA
TTCCAGAACCAGAACCAGAACCAGAACCAAAATCAAACTAAGACTCCTTACTCACAA
CAAAATTTTAATTTGTCTCCTGCAAAACAACTTAATTCAAATGTAGGTTCCATGCAT
TTATCACCTTTGAAAATCCATGCATTTATCACCTTTGAAAAACAATTACCAAPCAC
TCCCACAAAGCAACCCCCTGTCACCATTCAACAGCATCAACCTGAATTACAAAACAC
TTACCAACAAATTAAACACATTCAACAGCAACAGCAAATGCCGACGCCGAATCAAAA
AGACTTCATTGTTACCACCAGGTGAATTGGACAATTACTGGACAGGACCGGATGAGG
ATAAGATATATACTTGTACTTACAAAAACTGTGGAAAAAATTCACTCGTCGATACA
ATGTGCGAAGTCATATTCAGACACATTTAAGTGATCGTCCATTTGGTTGTCAATTCT
GTCCGAAAAGATTTGTCAGACAACATGATTTGAATCGTCACGTGAAAGGGCACATTG
 
AAGCTAGATATAGCAAATGTCCTTGTGGTAAAGAGTTTGCTAGATTAGATGCATTAA
GAAAACATCAGGATCGAAATATTTGTGTTGGTGGGAATAAAAATGTTATTAGTAAAC
CCACAAAGAAAAAGGGAACTAATAACACTCAACAACAATTGCTTAAAACAGATACAG
TGGTTGAGAGGATAGAAAAACAGTTGCTACAGGAAGATAAGAGTGTTACTGA
GGAGTTTTTAATGTTGCAATAG
**SEQ ID NO 16:**
   >gi|168487564|Iref|XM_707238.|I *Candida albicans* SC5314 hypothetical transcription factor (Ca019.6124) partial mRNA 
ATGCATTGGAAATTTCTGAACTTTCGAAAGTACCATCTTTCTTTCCATTTAAATTTATT
TGATCTTTCTCTCTTTTTATTTCCTTTTACTGCTTTCCCATCCTATATATTTGTTTCT
TTAATCAAGTCCATTCATTCAGAAGCACACAACCAAGTCTTATAATGAATAAATTTGAC
TTGTTTGATGATTACTCGACCAAAGGGTCAACCATCCCTCTTCCTAATGAAAATTTTGA
CCAATTATTTTTAAGTTCCGAAGCCAATGATATGGAATTTTTGTTTAATGAAACATTGA
TGGGATTACAAGATTTAGATGTTCCTTCTGGTTATGGAATTCCCCAAAATACCATTAAT
AACGATTTCCAACACACACCAAATAAGTCAAAATCTCACAGTAGACAATACAGTGGAAC
AGCAATTTTTGGTTTTGCTGACCATAATAAAGATTTGTCCATTAACGGTGTCAACAATG
ACTTGTGCAAACAATCCAATAAAGCCATCAATACACAATCGGTTTCACCTGGTGAGTTG
TTGAAACGTTCAAGAGGTTCTCAAACACCAACACCAACATCAGCACTACCAGACACTGC
CCAAGATATTTTAGATTTAATTTTGAGGAAAAACCAATATTGTTGCTTGAGGAGGATG
AGCTTGAGGAAGAAAAACATAAACAACAACAAAGAATGATGACACAGCTGTCACCTTTG
AAGAGAGTAACCACTCCTAGTCAATCACCATTTGTTCAACAGCCACAAACCATGAAACA
AAGAAAGCCACACAAGAAGACAAACGAGTACATTGTTGCCAATGAAAATCCCAATTCGT
ACAAATTTCCACCATCTCCATCTCCGACTGCCAAACGCCAGCAGTATCCACCATCATCT
CCAATACCATACAATCCAAAATCAGATTCAGTTGGTGGCAATTCTTACTCTGCAAAATA
CTTGCAGTCTTTAAACAAAACCCAGCAAATTGAATATGTGGATGATATTGAGCCACTTT
TACAGGAAGATAATAACAATATGAAATATATTCCAATTCCCGTACAAGAGCCAATGCTG
TATCAAAAACAGAAACCAGTGACACCTCCATTACAGTCACAAAATGATTCTCAACAATT
AGAACCATTGAAGACACCACAACCACAACCAAAACAACAACAACAGCAACAGCAACCAA
ATAATGAACAGGATAAAGAATTTACTGCTAATATCAATTTCAACACCTTCTTACCTCCA
CCTACTCCGCCTAATTTGATTAATGGATCTCCCGATTGGAATTCATCGCCAGAACCACA
TTCTCCTTCCCCAGGAAGATTGCAACCACCGCAACAGATATCCCCAATTCACCAGAATT
TAGGTGCCATGGGTAATAATATCAACTTTTATACACCCATGTATTACGAGCTTCCAGTA
 
CAAGCTGAACAACCGCAACCACAACCACAACCACATCAACAGCAACATCAACAGCAACA
GCATCAACCTGAATTACAAAACACTTACCAACAAATTAAACACATTCAACAGCAACAGC
AAATGCTACAACATCAGTTTCACAACCAAAACAATCAACTCCGCCAACAACACCCAAAT
CAATTCCAGAACCAGAACCAGAACCAGAACCAAAATCAAACTAAGACTCCTTACTCACA
ACAAAGTCAATTCTCACCTACACATTCCAATTTTAATTTGTCTCCTGCAAAACAACTTA
ATTCAAATGTAGGTTCCATGCATTTATCACCTTTGAAAAACAATTACCAAACACTCCC
ACAAAGCAACCCCCTGTCACCATTGAATGGAGTCCAGTTATATCACCAAACTCAAAGCA
ACCATTACACAAACAAATCAAAGAATCATCACCACGACGCCGAATCAAAAGACTTCAT
TGTTACCACCAGGTGAATTGGACAATTACTGGACAGGACCGGATGAGGATAAGATATAT
ACTTGTACTTACAAAAACTGTGGAAAAAATTCACTCGTCGATACAATGTGCGAAGTCA
TATTCAGACACATTTAAGTGATCG
TCCATTTGGTTGTCAATTCTGTCCGAAAAGATTTGTCAGACAACATGATTTGAATCGTC
ACGTGAAAGGGCACATTGAAGCTAGATATAGCAAATGTCCTTGTGGTAAAGAGTTTGCT
AGATTAGATGCATTAAGAAAACATCAGGATCGAAATATTTGTGTTGGTGGAATAAAAA
TGTTATTAGTAAACCCACAAAGAAAAAGGAACTAATAACACTCAACAACAATTGCTTA
AAACAGATACAGTGGTTGAGAGGATAGAAAAACAGTTGCTACAGGAAGATAAGAGTGTT
ACTGAGGAGTTTTTAATGTTGCAATAG
**SEQ ID NO 17:**
   >*C. tropicalis* Ace2 from whole genomic DNA sequence. Assembled manually (17146-19101) 
ATCATGGATAAGTTTGATTTATTTAGTGACTTTTCAAGTAGACCTTCAACTATACCTT
TACCTAGTGATAATTTCGATCAATTTTTATTAACTTCAGAACCAAATGATATTGATTT
CTTATTTAACGAAACTTTAAATGGTTTACAAGATTTAGATGTTCCTTCTGGTTACGGG
TTTAACCAACAACAAGCACCATCACAACAACAACAGCCATATCATTCACCTCATAGAT
CTAAAACACATAGTCGACAATATAGTGGTACTGCAATTTTTGGTTTCACAGAACATAC
AAGAGACTTATCTATAAACGGAATTAATAATGACTTGTATAAACAATATGTGAATAAA
GGACCTGAAAATAATTCTATTTCACCAAACGATTTATTAAGAGAAACCAATAATCATC
AACAAAATCCAATACCAACACAAGATATTTTAAATTTCAATTTTGATGAAAAACCAAT
ACTTTTATTAGAACAAGATGAGTTGGAAGATGAGAAACATTATTCGGCTAAATATTTA
CAATCCTTACAAAAATCACAACCACAGGAATATGTTGATGATATTGGTCCATTATTAC
AAGGAGATGAATTGTCTGATATTAAGTATATCCCCATTCCCGTTCAGGAACCAGTTGT
GTTTGATAAACAAGAAAAAAGGAATTCAGCCCCATTGGATCAATCAGAAGAATCAAAA
 
CAACCATCAAGAAAAGAACAATCTGACCAACAACAGGTACCACAACCAGTACCACAAC
CAGCAGCAGCACCACCACAACAGAAACAACAAACATTTCCAGGAAATTTCAATTTCAA
TACATATTTACCACCTCCTTCTTCACCACCACCTAATTTGAGTAATGGGTCTCCTGAC
TGGAATAGCTCTCCAGAACCACAATCTCCTTCGCCAAGTAGATTACAACCAAATCAAC
AAATTTCGCCAGTTCATCAAAATTTACGTGCCATGGGTAGCAATGTGAATTTTTATAA
TCCTGTGTATTATGAACAACATCCTCAACAACCTCCACCTTCACTACCACCTCATTCT
CAACCTTCTCAGCAACTGCAACTGCCTGGAATGCGTCAGACTTACGAACAAATCAAAC
AAATTCAACAACAACAACAAATGTTGCAACAACAATTTCACACTCAACAACAAACTCA
ACAACAACTTCAATCATCACCAATTTATTCCACTTTGAATTCATCACCAATTAAACAA
TTTAATCCTGATACAGTGAATAACTTACAAATATTTCACCTTTGAAAAACCAATTACC
AAGTACACCAACTAAACAACAAAATTCCCAAGTTACTATTGAATGGAGCCCTGTTATT
TCACCAAATTCCAAAGCATCATTATCTAAACAACTCAAAGAATCATCATTACAAACGT
CGCCACGTAGAAGAATTAAAAAGACTTCATTATTGCCACCTGGAGAACTTGATAATTA
TTGGATTGGACCCGATGAAAATAAAATTTATACATGTACTTATAAAAACTGTTTTAAA
AAATTTACAAGAAGATATAATGTTAGAAGTCATATTCAAACTCATTTAAGTGATAGAC
CATTTGGATGTCAATTTTGTCCTAAAAGATTTGTTCGACAACATGATTTAAATCGTCA
TGTTAAAGGACATATTGAAGCAAGATATAGTAAATGTCCATGTGGTAAAGAATTT
GCAAGATTAGATGCTTTAAGAAAACATCAAGATCGAAATATTTGTATTGGTGG
TAACAAA
**SEQ ID NO 18:**
   > gi1460199391emb1AJ630371.11 *Candida glabrata* Ace2 gene for transcription factor 
ATGAATACTTTCCAGGCGGATTGGGGAGAGATTCCTCAAATGCCAAAGGACCAAGTGT
TCACGCCCCAGGACCAACTAACGAATTACAACGATAACATGATGGACAACTTGCTTGA
CTTCAATTATAACGATGTTGATGCTCTCCTGTCGGAGGAGTTGAAGGACTTAGATATA
CCATTGGCGCCTTCGCCTAGAGACTTGAATATGAATGCCGAACAAAGTCTCAATTGGA
TGCAGGATATACAGGGACACAGGTCCAATAAACCTTCGATGTCGCATAAGAGAGGAAT
GAGTGGTACCGCAATATTTGGCTTCAAAAATCATAACAAAACCCTCAGCATTGCTAGT
TTTAGCAAGAATACAGACATTATCAATGAAGCTGAGAACGAGAATGTAAAAGGAAACA
CAGATAACCAGAATGGCTTTGTATTAAGCCAAGTTCTTCTGAAACAGCAGGAAGAGCT
TCGATTAGCTTTAGAAAAACAGAAAGAAGTAAATAGAAACTTAGAGCGGCAACTGCGA
GAAAATAGATTACAACAAGAACATATACAGCGTGTGCTTCACGATCAGGAAGCTGTAA
 
CAAGTCAATTAACAGCTCAAAACGTAACTGAATCGCCATCAAAACAAAGAAGTCCAAC
CAAATATCAAGGAGATGACGCCATCATAGTTACAAAAAACTCATCTTCTGGCGGCTAT
GTTTTCCCACCTCCACCTCGAGTTACATTAAATAACGAAGCTGTTACTCCACCGCTAT
CATTCTCAAGGTTTAGCAATATTGATCAAATGGAATCTTCAGATCCACTCAATTACCT
GCAACCAAATGCTGATTTCACAGAGGCTTATGCATCACACAAGACACCCGAATCATCA
TTTGGAAAGGAACATGCTTCAGTGCTTTCAACATCGGAATTTTTGAGACCTACCAATG
CTGCTAGAGAATCATCATCAAAGGCAATGTATAGCTCACCAAACAGTATGATTTCACC
TCATAGGAAAAAGATTCTGTTCTCTCAACAGTTTCTACTATTTTACAACCCCAAGAT
GACTATCAGAACACTGCAAGTCCGCCTTCGCAAATGTTGAACTTAGAAGAAGCCAATT
TAGAGAATGAACAAAGAACAATGGGAAAATGCTACGTGCACCAGTAGAAATTATGCC
AACTATACCGGGATCAAAAAATAATACTCCCATGACTGCTAATAAGTCTGGATTCATG
CCTCAGAAACATACTTTCCAGCAT
ACTCCTGTGAAAGCGAAAAATAATGTGGACATGAACGAGAGATCATTGGTACGGCCGG
AGCTATCAGGAACACCACTCAATAAGAGCGTACAAAATGGAATGCATTTTAGAAGA
GGACGACAGTAATATATTGCACCATATATCAGAAATACCGCAAGGATCAACATCACAT
AATAATACCACCGCTGGCGATGATTCAAATGTGGACAATAGGTTACAATTTTCTAATA
CCGAAAGTAGTCCAAGCCGGCAAAGGAAAAACCTACTACATTACCACCAGGTTATAT
TGATCGATTTGTTAAAGAACTACCTGACAAGAATTTTGAATGTTTATTTCCTAACTGT
GGTAAATTTTTTAGGAGAAGGTATAATATAAAGTCCCATATTCAAACACATCTAGAAG
ATAAACCATATAAGTGTGACTTTGAGGGATGCACTAAAGCGTTTGTGAGGAATCATGA
CCTTGCTAGACACAAGAAGACTCATGATAAGCATTTTTCTTGTCCCTGCGGAAAAAA
TTCAGCTCAGAACAATCAATGATGAAGCACAAGAATAGGCATAACTGCACTGGCCCTG
CCAGAGTTCCTGATTCCAAAATGGTATCCAAGTCTCCTAGAAAACAATCTTCTCCCAC
TAAACTTTCTTCAGCGATAATGAATAGCCCCATAAAGGAAAATTATCTGAAAGAAAAT
ACCAACCTACATATCGATCAATTAAGAATGGATCCTAAAATGAGAAATGCACTAGAGG
ATGGGGGCTATTGAAGCCAGTTGAGAGAACTGAAGCAATGGCTTTTCCATCTCCACT
ATCGGGATATAGTGATTTGGGTTCCCCTTTTAGAGATTTAGGACGATTGAGGAGTAG
**SEQ ID NO 19:**
   >gi|50294246|ref|XM 449535.1| *Candida glabrata* CBS138 hypothetical protein (CAGL0M0323g) partial mRNA ATGAATACTTTCCAGGCGGATTGGGAGAGATTCCTCAAATGCCAAAGGACCAAGTGTT 
CACACCCCAGGACCAACTAACGAATTACAACGATAACATGATGGACAACTTGCTTGACT
TCAATTATAACGATGTTGATGCTCTCCTGTCGGAGGAGTTGAAGGACTTAGATATACCA
TTGGCGCCTTCGCCTAGAGATCTGAATATGAATGCCGAACAAAGTCTCAATTGGATGCA
GGATATACAGGGACACAGGTCAAATAAACCTTCGATGTCGCATAAGAGAGGAATGAGTG
GTACCGCAATATTTGGCTTCAAAAATCATAACAAAACCCTCAGCATTGCTAGTTTTAGC
AAGAATACAGACATTATCAATGAAGCTGAGAACGAGAATGTAAAAGGAAACACAGATAA
CCAGAATGGCTTTGTATTAAGCCAAGTTCTTCTGAAACAGCAGGAAGAGCTTCGATTAG
CTTTAGAAAAACAGAAAGAAGTAAATAGAAACTTAGAGCGGCAACTGCGAGAAAATAGA
TTACAACAAGAACATATACAGCGTGTGCTTCACGATCAGGAAGCTGTAACAAGTCAATT
AACAGCTCAAAACGTAACTGAATCGCCATCAAAACAAAGAAGTCCAACCAAATATCAAG
GAGATGACGCCATCATAGTTACAAAAAACTCATCTTCTGGCGGCTATGTTTTCCCACCT
CCACCTCGAGTTACATTAAATAACGAAGCTGTTACTCCACCGCTATCATTCTCAAGGTT
TAGCAATATTGATCAAATGGAATCTTCAGATCCACTCAATTACCTGCAACCAAATGCTG
ATTTCACAGAGGCTTATGCATCACACAAGACACCCGAATCATCATTTGGAAAGGAGCAT
GCTTCAGTGCTTTCAACATCGGAATTTTTGAGACCTACCAATGCTGCTAGAGAATCATC
ATCAAAGGCAATGTATAGCTCACCAAACAGTATGATTTCACCTCATAGGAAAAAAGATT
CTGTTCTCTCAACAGTTTCTACTATTTTACAACCCCAAGATGACTATCAGAACACTGCA
AGTCCGCCTTCGCAAATGTTGAACTTAGAAGAAGCCAATTTAGAGAATGAACAAGAACA
ATGGGAAAATGCTACGTGCACCAGTAGAAATTATGCCAACTATACCGGATCAAAAAAT
AATACTCCCATGACTGCTAATAAGTCTGGATTCATGCCTCAGAAACATACTTTCCAGCA
TACTCCTGTGAAAGCGAAAAATAATGTGGACATGAACGAGAGATCATTGGTACGGCCGG
AGCTATCAGGAACACCACTCAATAAGAGCGTACAAAATGGAATGCATTTTAGAGAAGAG
GACGACAGTAATATATTGCACCATATATCAGAAATACCGCAAGGATCAACATCACATAA
TAATACCACCGCTGGCGATGATTCAAATGTGAGCAATAGGTTACAATTTTCTAATACCG
AAAGTAGTCCAAGCCGGCAAAGGAAAAAGCCTACTACATTACCACCAGGTTATATTGAT
CGATATGTTAAAGAACTACCTGACAAGAATTTTGAATGTTTATTTCCTAACTGTGGTAA
ATTTTTTAGGAGAAGGTATAATATAAAGTCCCATATTCAAACACATCTAGAAGATAAAC
CATATAAGTGTGACTTTGAGGGATGCACTAAAGCGTTTGTGAGGAATCATGACCTTGCT
AGACACAAGAAGACTCATGATAAGCATTTTTCTTGTCCCTGCGGAAAAAATTCAGCTC
AGAACAATCAATGATGAAGCACAAGAATAGGCATAACTGCACTGGCCCTGCCAGAGTTC
CTGATTCCAAAATGGTATCCAAGTCTCCTAGAAAACAATCTTCTCCCACTACTTTCTTC
AGCGATAATGAATAGCCCCATAAAGGAAAATTATCTGAAAGAAAATACCAACCTACATA
TCGATCAATTAAGAATGGATCCTAAAATGAGAAATGCACTAGAGGATGGGGGCTATTG
 
AAGCCAGTTGAGAGAACTGAAGCAATGGCTTTTCCATCTCCACTATCGGGATATA
GTGATTTGGGTTCCCCTTTTAGAGATTTAGGGACGATTGAGGAGTAG
**SEQ ID NO 20:** > ACF1 primer
   ATCAAAGAATCATCACCA
**SEQ ID NO 21:** > ACF2 primer
   AGACTTCATTGTTACCAC
**SEQ ID NO 22:** > ACF2b primer
   AAGACTTCATTGTTACCACC
**SEQ ID NO 23:** > ACF2c primer
   AGACTTCATTGTTACCACCAG
**SEQ ID NO 24:** > ACF3 primer
   CACCAGGTGAATTGG
**SEQ ID NO 25: >** ACR1 primer
   CATTGTATCGACGAGTG
**SEQ ID NO 26:** > ACR2 primer
   TGTATCGACGAGTGAAT
**SEQ ID NO** 27: > ACR3 primer
   TTCGCACATTGTATCG
**SEQ ID NO 28:** > ACR3b primer
   ACTTCGCACATTGTATCG
**SEQ ID NO 29:** > ACR3c primer
   TGACTTCGCACATTGTATCG
**SEQ ID NO 30:** > ACALB1 probe
   ATATCTTATCCTCATCCGGTCCT
**SEQ ID NO 31:** > ACALB2 probe
   AGGACCGGATGAGGATAAGATAT
**SEQ ID NO 32:**
   >CA3345/ACE2-Ace2 sequence generated for *C*. *albicans* 
AAACACTCCCACAAAGCAACCCCCTGTCACCATTGAATGGAGTCCAGTTATATCACCAA
ACTCAAAGCAACCATTACACAAACAAATCAAAGAATCATCACCACGACGCCGAATCAAA
AAGACTTCATTGTTACCACCAGGTGAATTGGACAATTACTGGACAGGACCGGATGAGGA
TAAGATATATACTTGTACTTACAAAAACTGTGGAAAAAATTCACTCGTCGATACAATG
TGCGAAGTCATATTCAGACACATTTAAGTGATCGTCCATTTGGTTGTCAATTCTGTCCG
AAAAGATTTGTCAGACAACATGATTTGAATCGTCACGTGAAAGGGCACATTGAAGCTAG
ATATAGCAAATGTCCTTGTGGTAAAGAG
SEQ ID NO 33:
   >CA16733/ACE2-Ace2 sequence generated for *C*. *albicans* 
AAACACTCCCACAAAGCAACCCCCTGTCACCATTGAATGGAGTCCAGTTATATCACCAA
ACTCAAAGCAACCATTACACAAACAAATCAAAGAATCATCACCACGACGCCGAATCAAA
AAGACTTCATTGTTACCACCAGGTGAATTGGACAATTACTGGACAGGACCGGATGAGGA
TAAGATATATACTTGTACTTACAAAAACTGTGGAAAAAATTCACTCGTCGATACAATG
TGCGAAGTCATATTCAGACACATTTAAGTGATCGTCCATTTGGTTGTCAATTCTGTCCG
AAAAGATTTGTCAGACAACATGATTTGAATCGTCACGTGAAAGGCACATTGAAGCTAG
ATATAGCAAATGTCCTTGTGGTAAAGA
**SEQ ID NO 34:**
   >CA1899/ACE2-Ace2 sequence generated for *C. albicans* 
AAACACTCCCACAAAGCAACCCCCTGTCACCATTGAATGGAGTCCAGTTATATCACCAA
ACTCAAAGCAACCATTACACAAACAAATCAAAGAATCATCACCACGACGCCGAATCAAA
AAGACTTCATTGTTACCACCAGGTGAATTGGACAATTACTGGACAGGACCGGATGAGGA
TAAGATATATACTTGTACTTACAAAAACTGTGGAAAAAATTCACTCGTCGATACAATG
TGCGAAGTCATATTCAGACACATTTAAGTGATCGTCCATTTGGTTGTCAATTCTGTCCG
AAAAGATTTGTCAGACAACATGATTTGAATCGTCACGTGAAAGGGCACATTGAAGCTAG
ATATAGCAAATGTCCTTGTGGTAAAGAGTT
**SEQ ID NO 35:**
   >CA1912/ACE2-Ace2 sequence generated for *C*. *albicans* 
AAACACTCCCACAAAGCAACCCCCTGTCACCATTGAATGGAGTCCAGTTATATCACCAA
ACTCAAAGCAACCATTACACAAACAAATCAAAGAATCATCACCACGACGCCGAATCAAA
AAGACTTCATTGTTACCACCAGGTGAATTGGACAATTACTGGACAGGACCGGATGAGGA
TAAGATATATACTTGTACTTACAAAAACTGTGGAAAAAATTCACTCGTCGATACAATG
TGCGAAGTCATATTCAGACACATTTAAGTGATCGTCCATTTGGTTGTCAATTCTGTCCG
AAAAGATTTGTCAGACAACATGATTTGAATCGTCACGTGAAAGGGCACATTGAAGCTAG
ATATAGCAAATGTCCTTGTGGTAAAGAG
**SEQ ID NO 36:**
   >CA2312/ACE2-Ace2 sequence generated for *C*. *albicans* 
AAACACTCCCACAAAGCAACCCCCTGTCACCATTGAATGGAGTCCAGTTATATCACCAA
ACTCAAAGCAACCATTACACAAACAAATCAAAGAATCATCACCACGACGCCGAATCAAA
AAGACTTCATTGTTACCACCAGGTGAATTGGACAATTACTGGACAGGACCGGATGAGGA
TAAGATATATACTTGTACTTACAAAAACTGTGGAAAAAATTCACTCGTCGATACAATG
TGCGAAGTCATATTCAGACACATTTAAGTGATCGTCCATTTGGTTGTCAATTCTGTCCG
AAAAGATTTGTCAGACAACATGATTTGAATCGTCACGTGAAAGGGCACATTGAAGCTAG
ATATAGCAAATGTCCTTGTGGTAAAGAGT
**SEQ ID NO 37:**
   >CA2688/ACE2-Ace2 sequence generated for *C*. *albicans* 
AAACACTCCCACAAAGCAACCCCCTGTCACCATTGAATGGAGTCCAGTTATATCACCAA
ACTCAAAGCAACCATTACACAAACAAATCAAAGAATCATCACCACGACGCCGAATCAAA
 
AAGACTTCATTGTTACCACCAGGTGAATTGGACAATTACTGGACAGGACCGGATGAGGA
TAAGATATATACTTGTACTTACAAAAACTGTGGAAAAAATTCACTCGTCGATACAATG
TGCGAAGTCATATTCAGACACATTTAAGTGATCGTCCATTTGGTTGTCAATTCTGTCCG
AAAAGATTTGTCAGACAACATGATTTGAATCGTCACGTGAAAGGGCACATTGAAGCTAG
ATATAGCAAATGTCCTTGTGGTAAAGAGTT
**SEQ ID NO 38:**
   >CA2701/ACE2-Ace2 sequence generated for *C. albicans* 
AAACACTCCCACAAAGCAACCCCCTGTCACCATTGAATGGAGTCCAGTTATATCACCAA
ACTCAAAGCAACCATTACACAAACAAATCAAAGAATCATCACCACGACGCCGAATCAAA
AAGACTTCATTGTTACCACCAGGTGAATTGGACAATTACTGGACAGGACCGGATGAGGA
TAAGATATATACTTGTACTTACAAAAACTGTGGAAAAAATTCACTCGTCGATACAATG
TGCGAAGTCATATTCAGACACATTTAAGTGATCGTCCATTTGGTTGTCAATTCTGTCCG
AAAAGATTTGTCAGACAACATGATTTGAATCGTCACGTGAAAGGGCACATTGAAGCTAG
ATATAGCAAATGTCCTTGTGGTAAAGA
**SEQ ID NO 39:**
   >CA15640/ACE2-Ace2 sequence generated for *C*. *albicans* 
AAACACTCCCACAAAGCAACCCCCTGTCACCATTGAATGGAGTCCAGTTATATCACCAA
ACTCAAAGCAACCATTACACAAACAAATCAAAGAATCATCACCACGACGCCGAATCAAA
AAGACTTCATTGTTACCACCAGGTGAATTGGACAATTACTGGACAGGACCGGATGAGGA
TAAGATATATACTTGTACTTACAAAAACTGTGGAAAAAATTCACTCGTCGATACAATG
TGCGAAGTCATATTCAGACACATTTAAGTGATCGTCCATTTGGTTGTCAATTCTGTCCG
AAAAGATTTGTCAGACAACATGATTTGAATCGTCACGTGAAAGGGCACATTGAAGCTAG
ATATAGCAAATGTCCTTGTGGTAAAG
**SEQ ID NO 40:**
   >T94/ACE2 - Ace2sequence generated for *C*. *tropicalis*

AAGATACACCAACTAAACAACAAAATTCCCAAGTTACTATTGAATGGAGCCCTGTTATT
TCACCAAATTCCAAAGCATCATTATCTAAACAACTCAAAGAATCATCATTACAAACGTC
GCCACGTAGAAGAATTAAAAAGACTTCATTATTGCCACCTGGAGAACTTGATAATTATT
GGATTGGACCCGATGAAAATAAAATTTATACATGTACTTATAAAAACTGTTTTAAAAAA
TTTACAAGAAGATATAATGTTAGAAGTCATATTCAAACTCATTTAAGTGATAGACCATT

TGGATGTCAATTTTGTCCTAAAAGATTTGTTCGACAACATGATTTAAATCGTCATGTTA
AAGGACATATTGAAGCAAGATATAGTAAA

## Claims

1. Use of a diagnostic kit for the discrimination of *Candida albicans,* the kit comprising an oligonucleotide probe capable of binding to at least a portion of the *Candida albicans* Ace2 gene or its corresponding mRNA, wherein the probe is selected from the group consisting of SEQ ID Nos: 3, 30, 31 and sequences of between 10 and 100 nucleotides in length which preferentially hybridize to the nucleotide sequences SEQ ID Nos: 3, 30, 31 and which are able to discriminate *C.albicans* from other yeast species, and sequences of between 10 and 100 nucleotides in length which are complementary thereto.

2. Use of a kit as claimed in any preceding claim, further comprising a primer for amplification of at least a portion of the Ace2 gene, the primer being a forward and a reverse primer for a portion of the Ace2 gene.

3. Use of a kit as claimed in any preceding claim wherein the kit comprises at least one forward *in vitro* amplification primer and at least one reverse *in vitro* amplification primer, the forward amplification primer being selected from the group consisting of SEQ ID Nos: 1, 20, 21 and 24, sequences of between 10 and 100 nucleotides in length which are complementary to sequences that preferentially hybridize to the nucleotide sequences SEQ ID Nos: 1, 20, 21 and24 and are able to discriminate *C.albicans* from other yeast species, and which can also act as a forward amplification primer;
and the reverse amplification primer being selected from the group consisting of SEQ ID Nos: 2, 25, 26 and 27, sequences of between 10 and 100 nucleotides in length which are complementary to sequences that preferentially hybridize to the nucleotide sequences SEQ ID Nos: 2, 25, 26 and 27, and are able to discriminate *C.albicans* from other yeast species, and which can also act as a reverse amplification primer.

4. Use of a kit as claimed in claim 3, wherein said forward primer sequence is selected from the group consisting of SEQ ID NOs:20 and 21, and said reverse primer sequence is selected from the group consisting of SEQ ID NOs:25 to 27.

5. Use of a nucleic acid molecule selected from the group consisting of: SEQ ID NOs 1, 2, 3, 21, 24, 25, 26, 27, 30 and 31, sequences of between 10 and 100 nucleotides in length which preferentially hybridize to the nucleotide sequences SEQ ID NOs 1, 2, 3, 21, 24, 25, 26, 27, 30 and 31, and are able to discriminate *C.albicans* from other yeast species, sequences of between 10 and 100 nucleotides in length which are complementary thereto and SEQ ID NO:20 for the discrimination of *Candida albicans.*

6. A method of discriminating *Candida albicans* in a test sample comprising the steps of:
(i) Mixing the test sample with at least one oligonucleotide probe capable of binding to at least a portion of the *Candida albicans* Ace2 gene or its corresponding mRNA under appropriate conditions;
(ii) hybridizing under high stringency conditions any nucleic acid that may be present in the test sample with the oligonucleotide to form a probe:target duplex;and
(iii) determining whether a probe:target duplex is present; the presence of the duplex positively identifying the presence of *Candida albicans* in the test sample, wherein the probe is selected from the group consisting of SEQ ID Nos: 3, 30, 31, sequences of between 10 and 100 nucleotides in length which preferentially hybridize to the nucleotide sequences SEQ ID Nos: 3, 30, 31 and are able to discriminate *C*. *albicans* from other yeast species, and sequences complementary thereto.

7. Use of a kit as claimed in any one of claims 1 to 4 or use of a nucleic acid molecule as claimed in claim 5: in a diagnostic assay to measure yeast titres in a patient; in a diagnostic assay to detect the presence of one or more of a yeast and/or fungal species; in a diagnostic assay to measure yeast contamination in an environment; or in the identification and/or characterization of one or more disruptive agents that can be used to disrupt the Ace2 gene function.

8. A method of assessing the efficacy of a treatment regime designed to reduce yeast titre in a patient comprising use of a kit as claimed in any one of claims 1 to 4 or use of a nucleic acid molecule as claimed in claim 5 at one or more key stages of the treatment regime, wherein the key stages comprise before treatment, during treatment and after treatment.

9. Use as claimed in claim 7, wherein the environment is a hospital, a food sample, an environmental sample e.g. water, an industrial sample such as an in-process sample or an end product requiring bioburden or quality assessment.

10. Use as claimed in claim 7, wherein the disruptive agent is selected from the group consisting of antisense RNA, PNA, siRNA.

## Patentansprüche

1. Verwendung eines Diagnose-Kits zur Unterscheidung von *Candida albicans*, das Kit umfassend eine Oligonucleotidesonde, die an wenigstens einen Teil des *Candida-albicans*-Ace2-Gens oder dessen entsprechender mRNA binden kann, wobei die Sonde ausgewählt ist aus der Gruppe bestehend aus SEQ ID NOs: 3, 30, 31 und Sequenzen mit einer Länge zwischen 10 und 100 Nucleotiden, die vorzugsweise zu den Nucleotid-Sequenzen SEQ ID NOs: 3, 30, 31 hybridisieren und die *C.albicans* von anderen Hefespezies unterscheiden können, und Sequenzen mit einer Länge zwischen 10 und 100 Nucleotiden, die dazu komplementär sind.

2. Verwendung eines Kits nach einem der vorstehenden Ansprüche, ferner umfassend einen Primer zur Amplifizierung von wenigstens einem Teil des Ace2-Gens, wobei der Primer ein Forward- und ein Reverse-Primer für einen Teil des Ace2-Gens ist.

3. Verwendung eines Kits nach einem der vorstehenden Ansprüche, wobei das Kit wenigstens einen Forward-*In*-*vitro*-Amplifizierungs-Primer und wenigstens einen Reverse*-In-vitro-*Amplifizierungs-Primer umfasst, wobei der Forward-Amplifizierungs-Primer ausgewählt ist aus der Gruppe bestehend aus SEQ ID NOs: 1, 20, 21 und 24, Sequenzen mit einer Länge zwischen 10 und 100 Nucleotiden, die komplementär sind zu Sequenzen, die vorzugsweise zu den Nucleotid-Sequenzen SEQ ID NOs: 1, 20, 21 und 24 hybridisieren und *C.albicans* von anderen Hefespezies unterscheiden können und die auch als Forward-Amplifizierungs-Primer wirken können; und der Reverse-Amplifizierungs-Primer ausgewählt aus der Gruppe bestehend aus SEQ ID NOs: 2, 25, 26 und 27, Sequenzen mit einer Länge zwischen 10 und 100 Nucleotiden, die komplementär sind zu Sequenzen, die vorzugsweise zu den Nucleotid-Sequenzen SEQ ID NOs: 2, 25, 26 und 27 hybridisieren und *C.albicans* von anderen Hefespezies unterscheiden können und die auch als Reverse-Amplifizierungs-Primer wirken können.

4. Verwendung eines Kits nach Anspruch 3, wobei die Forward-Primer-Sequenz ausgewählt ist aus der Gruppe bestehend aus SEQ ID NOs: 20 und 21 und die Reverse-Primer-Sequenz ausgewählt ist aus der Gruppe bestehend aus SEQ ID NOs: 25 bis 27.

5. Verwendung eines Nukleinsäuremoleküls, ausgewählt aus der Gruppe bestehend aus: SEQ ID NOs 1, 2, 3, 21, 24, 25, 26, 27, 30 und 31, Sequenzen mit einer Länge zwischen 10 und 100 Nucleotiden, die vorzugsweise zu den Nucleotid-Sequenzen SEQ ID NOs 1, 2, 3, 21, 24, 25, 26, 27, 30 und 31 hybridisieren und *C.albicans* von anderen Hefespezies unterscheiden können, Sequenzen mit einer Länge zwischen 10 und 100 Nucleotiden, die dazu komplementär sind, und SEQ ID NO: 20 zur Unterscheidung von *Candida albicans.*

6. Verfahren zur Unterscheidung von *Candida albicans* in einer Testprobe, umfassend die folgenden Schritte:
(i) Mischen der Testprobe mit wenigstens einer Oligonucleotidsonde, die an wenigstens einen Teil des *Candida-albicans*-Ace2-Gens oder dessen entsprechender mRNA unter geeigneten Bedingungenbinden kann;
(ii) Hybridisieren unter hoch stringenten Bedingungen von Nukleinsäure, die möglicherweise in der Testprobe vorhanden ist, mit dem Oligonucleotid, um ein Sonden-Target-Duplex zu bilden; und
(iii) Bestimmen, ob ein Sonden-Target-Duplex vorhanden ist, wobei das Vorhandensein des Duplex das Vorhandensein von *Candida albicans* in der Testprobe eindeutig identifiziert, wobei die Sonde ausgewählt ist aus der Gruppe bestehend aus SEQ ID NOs: 3, 30, 31, Sequenzen mit einer Länge zwischen 10 und 100 Nucleotiden, die vorzugsweise zu den Nucleotid-Sequenzen SEQ ID NOs: 3, 30, 31 hybridisieren und *C*. *albicans* von anderen Hefespezies unterscheiden können, und Sequenzen, die dazu komplementär sind.

7. Verwendung eines Kits nach einem der Ansprüche 1 bis 4 oder Verwendung eines Nukleinsäuremoleküls nach Anspruch 5: in einem diagnostischen Assay, um Hefetiter in einem Patienten zu messen; in einem diagnostischen Assay, um das Vorhandensein von einer oder mehreren Hefe- und/oder Pilzspezies nachzuweisen; in einem diagnostischen Assay, um Hefekontamination in einer Umgebung zu messen, oder bei der Identifizierung und/oder Charakterisierung von einem oder mehreren disruptiven Mitteln, die zur Unterbrechung der Funktion des Ace2-Gens verwendet werden können.

8. Verfahren zur Beurteilung der Wirksamkeit eines Behandlungsregimes zur Reduzierung von Hefetiter in einem Patienten, umfassend die Verwendung eines Kits nach einem der Ansprüche 1 bis 4 oder die Verwendung eines Nukleinsäuremoleküls nach Anspruch 5 in einer oder mehreren Hauptstufen des Behandlungsregimes, wobei die Hauptstufen Stufen vor der Behandlung, während der Behandlung und nach der Behandlung umfassen.

9. Verwendung nach Anspruch 7, wobei die Umgebung ein Krankenhaus, eine Lebensmittelprobe, eine Umgebungsprobe, z. B. Wasser, eine industrielle Probe wie einer In-Prozess-Probe oder ein Endprodukt dessen mikrobielle Belastung oder dessen Qualität beurteilt werden muss, ist.

10. Verwendung nach Anspruch 7, wobei das disruptive Mittel ausgewählt ist aus der Gruppe bestehend aus Antisense-RMA, -PNA, -siRNA.

## Revendications

1. Utilisation d'un kit de diagnostic pour la discrimination de *Candida albicans,* ledit kit comprenant une sonde oligonucléotidique capable de lier au moins une partie du gène Ace2 de *Candida albicans* ou de son ARN messager correspondant, la sonde étant sélectionnée dans le groupe constitué des SEQ ID N° 3, 30, 31 et des séquences ayant entre 10 et 100 nucléotides de long s'hybridant de préférence aux séquences nucléotidiques SED ID N° 3, 30, 31, et capables de discriminer *Candida albicans* d'autres espèces de levures, et des séquences ayant entre 10 et 100 nucléotides de long, complémentaires avec celles-ci.

2. Utilisation d'un kit selon la revendication 1, comprenant en outre une amorce pour l'amplification d'au moins une partie du gène Ace2, l'amorce étant une amorce sens (*forward*) et antisens (*reverse*) pour une partie du gène Ace2.

3. Utilisation d'un kit selon l'une des revendications précédentes, le kit comprenant au moins une amorce sens d'amplification *in vitro* et au moins une amorce antisens d'amplification *in vitro,* ladite amorce sens d'amplification étant sélectionnée dans le groupe constitué des SEQ ID N° 1, 20, 21 et 24, de séquences ayant entre 10 et 100 nucléotides de long complémentaires avec des séquences s'hybridant de préférence aux séquences nucléotidiques SED ID N° 1, 20, 21 et 24, et capables de discriminer *Candida albicans* d'autres espèces de levures, et pouvant également faire office d'amorce sens d'amplification ; et ladite amorce antisens d'amplification étant sélectionnée dans le groupe constitué des SEQ ID N° 2, 25, 26 et 27, de séquences ayant entre 10 et 100 nucléotides de long complémentaires avec des séquences s'hybridant de préférence aux séquences nucléotidiques SED ID N° 2, 25, 26 et 27, et capables de discriminer *Candida albicans* d'autres espèces de levures, et pouvant également faire office d'amorce antisens d'amplification.

4. Utilisation d'un kit selon la revendication 3, ladite séquence d'amorce sens étant sélectionnée dans le groupe constitué de SEQ ID N° 20 et 21 et ladite séquence d'amorce antisens étant sélectionnée dans le groupe composé des SEQ ID N° 25 à 27.

5. Utilisation d'une molécule d'acide nucléique sélectionnée dans le groupe composé des SEQ ID N° 1, 2, 3, 21, 24, 25, 26, 27, 30 et 31, de séquences ayant entre 10 et 100 nucléotides de long s'hybridant de préférence aux séquences nucléotidiques SED ID N° 1, 2, 3, 21, 24, 25, 26, 27, 30 et 31, et capables de discriminer *Candida albicans* d'autres espèces de levures, et de séquences ayant entre 10 et 100 nucléotides de long complémentaires avec celles-ci, et de la SEQ ID N° 20 pour la discrimination de *Candida albicans*.

6. Procédé de discrimination de *Candida albicans* dans un échantillon à tester, comprenant les étapes suivantes :
(i) mélanger l'échantillon à tester avec au moins une sonde oligonucléotidique capable de lier au moins une partie du gène Ace2 de *Candida albicans* ou de son ARN messager correspondant dans des conditions adaptées ;
(ii) hybrider à l'oligonucléotide dans des conditions de forte stringence tout acide nucléique potentiellement présent dans l'échantillon à tester, de façon à former un duplex sonde-cible ; et
(iii) déterminer la présence ou non d'un duplex sonde-cible, la présence du duplex permettant d'identifier positivement la présence de *Candida albicans* dans l'échantillon à tester, la sonde étant sélectionnée dans le groupe constitué des SEQ ID N° 3, 30, 31, de séquences ayant entre 10 et 100 nucléotides de long s'hybridant de préférence aux séquences nucléotidiques SED ID N° 3, 30, 31, et capables de discriminer *Candida albicans* d'autres espèces de levures, et de séquences complémentaires avec celles-ci.

7. Utilisation d'un kit selon l'une quelconque des revendications 1 à 4, ou utilisation d'une molécule d'acide nucléique selon la revendication 5, dans un test diagnostique destiné à mesurer des titres de levure chez un patient ; et dans un test diagnostique destiné à détecter la présence d'une ou de plusieurs espèces de levures et/ou fongiques ; dans un test diagnostique destiné à mesurer une contamination par les levures dans un environnement ; ou pour identifier et/ou caractériser un ou plusieurs agent(s) perturbateur(s)(s) pouvant être utilisé(s) pour déstabiliser le fonctionnement du gène Ace2.

8. Procédé d'évaluation de l'efficacité d'un régime de traitement conçu pour réduire le titre de levure chez un patient, comprenant l'utilisation d'un kit selon l'une quelconque des revendications 1 à 4, ou l'utilisation d'une molécule d'acide nucléique selon la revendication 5, à une ou plusieurs étapes clés du régime de traitement, lesdites étapes clés comprenant les étapes avant le traitement, pendant le traitement et après le traitement.

9. Utilisation selon la revendication 7, l'environnement étant un hôpital, un échantillon alimentaire, un échantillon environnemental tel que par ex. de l'eau, un échantillon industriel tel que par ex. un échantillon semi-fini ou un produit fini nécessitant une charge microbienne ou une évaluation de la qualité.

10. Utilisation selon la revendication 7, l'agent perturbateur étant sélectionné dans le groupe constitué d'ARN antisens, d'acide peptidonucléique et de petits ARN interférents (ARNsi).
